(19) Europäisches Patentamt
European Patent Office
Office européen des brevets

(11) **EP 3 642 326 B1**

(12) **FASCICULE DE BREVET EUROPEEN**

(45) Date de publication et mention de la délivrance du brevet:
**31.07.2024 Bulletin 2024/31**

(21) Numéro de dépôt: **18731126.1**

(22) Date de dépôt: **21.06.2018**

(51) Classification Internationale des Brevets (IPC):
***C12N 5/071*** (2010.01)    ***A61K 35/36*** (2015.01)

(52) Classification Coopérative des Brevets (CPC):
**C12N 5/0698; A61L 27/60; G01N 33/5088;**
C12N 2503/06

(86) Numéro de dépôt international:
**PCT/EP2018/066542**

(87) Numéro de publication internationale:
**WO 2018/234430 (27.12.2018 Gazette 2018/52)**

(54) **MODELES DE PEAU SENSIBLE RECONSTITUEE**

MODELLE VON REKONSTRUIERTER EMPFINDLICHER HAUT

MODELS OF RECONSTRUCTED SENSITIVE SKIN

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **22.06.2017   FR 1755697**

(43) Date de publication de la demande:
**29.04.2020   Bulletin 2020/18**

(73) Titulaire: **Laboratoires Expanscience**
**92048 Paris La Défense Cedex (FR)**

(72) Inventeurs:
 • **BELLEMERE, Gaëlle**
   **76420 Bihorel (FR)**
 • **BREDIF, Stéphanie**
   **28210 Croisilles (FR)**
 • **BOYER, Gaëtan**
   **28210 Coulombs (FR)**
 • **BAUDOUIN, Caroline**
   **78120 Rambouillet (FR)**

(74) Mandataire: **Regimbeau**
**20, rue de Chazelles**
**75847 Paris Cedex 17 (FR)**

(56) Documents cités:
WO-A1-2008/096868     WO-A1-2014/028734
WO-A1-2015/101677     WO-A1-2015/104413

 • HERNANDEZ-PIGEON H ET AL: "A new model of "fragile skin" in vitro on human keratinocytes and reconstructed epidermis", THE JOURNAL OF INVESTIGATIVE DERMATOLOGY : OFFICIAL JOURNAL OF THE SOCIETY FOR INVESTIGATIVE DERMATOLOGY AND THE EUROPEAN SOCIETY FOR DERMATOLOGICAL RESEARCH, ELSEVIER, US, vol. 134, no. Suppl. 1, 7 May 2014 (2014-05-07), XP002730023, ISSN: 0022-202X
 • MAIERDANJIANG WUFUER ET AL: "Skin-on-a-chip model simulating inflammation, edema and drug-based treatment", SCIENTIFIC REPORTS, vol. 6, no. 1, 21 November 2016 (2016-11-21), XP055435914, DOI: 10.1038/srep37471
 • N. CASTEX-RIZZI ET AL: "In vitro approaches to pharmacological screening in the field of atopic dermatitis", BRITISH JOURNAL OF DERMATOLOGY, vol. 170, 16 July 2014 (2014-07-16), UK, pages 12 - 18, XP055352641, ISSN: 0007-0963, DOI: 10.1111/bjd.13106
 • POUMAY Y ET AL: "A simple reconstructed human epidermis: preparation of the culture model and utilization in in vitro studies", ARCHIVES OF DERMATOLOGICAL RESEARCH, SPRINGER, INTERNATIONAL, BERLIN, DE, vol. 296, no. 5, 1 October 2004 (2004-10-01), pages 203 - 211, XP002510240, ISSN: 0340-3696, DOI: 10.1007/S00403-004-0507-Y

**(Cont. page suivante)**

- NETZLAFF F ET AL: "The human epidermis models EpiSkin^(R), SkinEthic^(R) and EpiDerm^(R): An evaluation of morphology and their suitability for testing phototoxicity, irritancy, corrosivity, and substance transport", EUROPEAN JOURNAL OF PHARMACEUTICS AND BIOPHARMACEUTICS, ELSEVIER SCIENCE PUBLISHERS B.V., AMSTERDAM, NL, vol. 60, no. 2, 1 July 2005 (2005-07-01), pages 167 - 178, XP027805122, ISSN: 0939-6411, [retrieved on 20050701]

**Description**

INTRODUCTION

La peau est un ensemble de cellules et de macromolécules regroupées sous forme d'un tissu résistant et souple, recouvrant la totalité du corps. Elle est formée de deux couches jointes : l'épiderme et le derme auxquelles on peut associer les tissus sous-cutanés.

L'épiderme dont le principal rôle est la protection du corps constitue la couche la plus superficielle de la peau et assure l'imperméabilité de la peau et sa résistance. On peut identifier dans celui-ci quatre couches cellulaires distinctes, une couche basale (*stratum basalis*), une couche épineuse (*stratum spinosum*), une couche granuleuse (*stratum granulosum*), et une couche cornée (*stratum corneum*). Si différents types cellulaires coexistent dans l'épiderme, les kératinocytes sont largement majoritaires (90 %). Leur activité caractéristique est la synthèse des kératines, des protéines fibreuses et insolubles dans l'eau qui représentent 95 % des protéines totales de l'épiderme. Les kératinocytes peuvent aussi produire de la filaggrine, comme présenté par la demande WO 2015/104413.

La peau a pour fonction principale d'établir une barrière de protection contre les atteintes de l'environnement tout en permettant certains échanges entre le milieu intérieur et le milieu extérieur. La fonction de barrière est assurée avant tout par la couche cornée (*stratum corneum*), laquelle rend la peau imperméable et hydrophobe, protégeant ainsi le derme d'une entrée massive d'eau. C'est également la couche cornée qui résiste aux agressions chimiques. Elle se compose de cellules, appelées cornéocytes, mortes et sans noyau, mais remplies de kératines et d'autres produits comme les lipides, les acides gras et les céramides. Les cornéocytes sont reliés par des jonctions serrées particulières, les cornéodesmosomes, formant une couche compacte dont la cohésion est encore renforcée par un ciment lipidique. Sous la couche granuleuse, les jonctions serrées dans la couche granuleuse participent aussi de la fonction de barrière de la peau (voir par exemple, Hogan et al., J Allergy, 2012 : 901940, 2012).

La peau dite sensible est un syndrome courant dans la population. Elle manque cependant d'une définition claire. Il s'agit en effet d'un syndrome déclaratif, dont les manifestations cliniques sont avant tout liées à des sensations d'inconfort (démangeaisons, picotements, brûlure, tiraillements...). En revanche, la peau sensible n'est la plupart du temps pas associée à des symptômes cliniques objectivement observables, même si elle est souvent associée à la peau rouge érythémateuse.

Au quotidien, la peau doit faire face à des attaques diverses. Elle est exposée, par exemple, à des agents chimiques comme le savon ainsi qu'à des stress physiques comme la friction avec les vêtements et l'exposition au soleil. La sensibilité cutanée semble être déclenchée lorsque la peau est soumise à différents types de stress, comme l'exposition au soleil, certains produits chimiques, certains facteurs physiques comme la laine, le froid, le vent, etc... Par ailleurs, un modèle de peau fragilisé par l'exposition à un stress chimique induit par du SDS, suivi d'un stress déshydratant, est proposé dans la demande WO 2015/101677 et l'article scientifique correspondant (Hernandez-Pigeon et al., The journal of investigative dermatology, 2014,134(3)). Les signes cliniques décrits ci-dessus apparaissent après exposition à ces facteurs de stress. Hormis ces manifestations consécutives à une exposition, aucune étude n'a réussi à démontrer de différence, histologique ou physiologique, entre une peau normale et une peau sensible en condition basale.

Bien que l'étiologie exacte de ce syndrome soit encore mal connue, il semblerait que des fonctions cutanées soit anormales ou déficientes chez les sujets à peau sensible. Selon les hypothèses les plus couramment admises, la peau sensible serait caractérisée par une fonction barrière altérée, une pénétration transcutanée accrue, une hyperréactivité inflammatoire et/ou vasculaire, ainsi que des variations dans la densité en fibres nerveuses et dans la réponse de celles-ci aux stimuli (Richters et al., Skin Pharmacol Physiol. 2015, 28(2) : 75-83). Cependant, les facteurs médiant le syndrome de la peau sensible n'ont pas été identifiés aujourd'hui. En outre, les mécanismes entraînant la sensibilité cutanée sont mal connus. L'hypothèse la plus souvent évoquée est celle d'une plus grande perméabilité de la peau sensible à la pénétration de certains types d'ingrédients, entraînant une réactivité cutanée. Le seuil de tolérance d'une peau sensible serait diminué, sans que cette hypothèse n'ait été totalement démontrée.

En l'absence de signes cliniques objectifs, les sujets ayant la peau sensible sont identifiés généralement par auto évaluation subjective d'une sensation de picotement ressentie, après application d'une solution d'acide lactique (Bererdesca et al., Int J Cosmet Sci. 2013, 35(1) : 2-8). Toutefois, ce type de test ne permet pas de définir objectivement en quoi la structure de la peau sensible diffère de celle de la peau normale. En effet, les différents mécanismes moléculaires et cellulaires mis en oeuvre dans la peau sensible ne sont toujours pas connus. Le diagnostic et le traitement en restent donc d'autant plus difficiles.

La préparation de modèle de peau est décrite notamment par Poumay et al. (Archives of dermatology research, 2004, 296(5), 203-211) ainsi que Netzlaff et al. (European journal of pharmaceutics and biopharmaceutics, 2005, 60(2), 167-178). Des modèles de peau, sains ou atteints par des maladies comme la dermatite atopique, soumis à un stress dans le but d'étudier la réponse inflammatoire produite ont aussi été proposé par Wufuer et al. (Scientific report, 2016, 6(1)) et Castex-Rizzi et al. (British journal of dermatology, 2014, 170, 12-18), ainsi que dans les demandes WO 2008/096868 et WO 2014/028734. Cependant, il existe toujours un besoin pour un modèle reproduisant les caractéristiques cliniques et structurelles de la peau sensible.

DESCRIPTION

L'invention est définie dans les revendications et tous les autres aspects ou modes de réalisation exposés ici le sont à titre indicatif.

L'invention a notamment pour objet un modèle de peau sensible reconstituée, ledit modèle étant susceptible d'être obtenu par un procédé comprenant des étapes de :

a) obtention d'un modèle de peau reconstituée à partir d'un échantillon cutané d'un sujet ; et

b) mise en contact dudit modèle de peau reconstituée de l'étape a) avec de l'acide lactique, la concentration d'acide lactique utilisée étant comprise entre 0,05 % et 5 % ;

ledit modèle exprimant au moins un marqueur biologique choisi dans le groupe des marqueurs de l'inflammation, des marqueurs de la barrière et des marqueurs de défense ; et dans lequel :

(i) l'expression du marqueur biologique choisi dans le groupe des marqueurs de l'inflammation est plus élevée que dans la peau normale, et/ou

(ii) l'expression du marqueur biologique choisi dans le groupe des marqueurs de la barrière est moins élevée que dans la peau normale ; et/ou

(iii) l'expression du marqueur biologique choisi dans le groupe des marqueurs de défense est moins élevée que dans la peau normale.

Les présents inventeurs ont créé un modèle de peau reconstituée qui reproduit les caractéristiques de la peau sensible telles qu'ils les ont identifiées à travers une étude clinique exhaustive.

La peau sensible est un syndrome mal connu car reposant pour une large part sur des critères subjectifs. Les inventeurs ont conduit une étude clinique qui leur a permis de caractériser les différents aspects cellulaires, moléculaires et physiologiques de la peau sensible. Ils ont notamment montré que la peau sensible chez l'enfant comme chez l'adulte est caractérisée par une forte induction des marqueurs de l'inflammation et une augmentation des pertes d'eau transcutanées, signes d'une altération de la fonction barrière.

Sur la base des résultats de cette étude clinique, les inventeurs ont mis au point un modèle de peau *in vitro* spécialisé permettant de reproduire les caractéristiques biologiques de la peau sensible, et pouvant notamment être utilisé pour cribler des actifs ou encore des formulations cosmétiques ou pharmacologiques d'intérêts.

Le modèle décrit ici a pour particularité de se rapprocher de la véritable peau sensible en ce qu'il comprend un profil de marqueurs de l'inflammation très comparable à celui identifié chez les sujets à peau sensible. Les mêmes cytokines sont notamment induites dans le présent modèle que chez lesdits sujets à peau sensible.

Le modèle de peau décrit est donc particulièrement simple à mettre en oeuvre. En outre, il ne nécessite pas d'utiliser une lignée cellulaire commerciale particulière, et s'avère versatile et adaptable.

Les aspects particuliers du modèle divulgué permettent d'étudier *in vitro* les particularités de la peau sensible. Les inventeurs ont notamment observé que l'application d'un stress exogène sur un modèle de peau reconstituée induisait des aspects physiologiques, cellulaires et moléculaires semblables à ceux observés chez les sujets ayant une peau sensible.

Par « sujet », on entend ici toute personne humaine, que ce soit un adulte ou un enfant. Par « enfant », on entend selon la divulgation un individu dont l'âge est inférieur ou égal à 16 ans. Sont ainsi compris dans la catégorie des enfants selon la divulgation, les nouveau-nés, dont l'âge est compris entre 0 et 1 mois, les nourrissons, qui ont entre 1 mois et 2 ans, et les enfants proprement dits, qui sont âgés d'au moins 2 ans. Un « nouveau-né », comme on l'entend ici, peut aussi bien être né à terme qu'être prématuré. Pour lever toute ambiguïté, le terme « enfant » utilisé dans la présente demande sans autre précision doit être entendu dans son acception la plus générale, c'est-à-dire comme se référant à une personne de 16 ans ou moins. Un « adulte » au sens de la présente demande est une personne qui n'est pas un enfant, autrement dit une personne âgée de plus de 16 ans.

Par exemple, le modèle tel que décrit peut être obtenu avec n'importe quel type de peau sensible, notamment quelle que soit l'origine ethnique ou géographique de la peau, ou encore le phototype de celle-ci. Elle peut ainsi être d'origine caucasienne, africaine, asiatique, sud-américaine, mélanésienne ou autre ; elle peut encore présenter un phototype I, II, III, IV, V ou VI, sans que cela n'affecte la divulgation. Celle-ci a en effet pour objet l'identification de marqueurs biologiques caractérisant n'importe quel type de peau sensible.

Un premier aspect de la divulgation a donc pour objet un modèle de peau sensible reconstituée, ledit modèle étant susceptible d'être obtenu par un procédé comprenant des étapes de :

a) obtention d'un modèle de peau reconstituée à partir d'un échantillon cutané d'un sujet ; et
b) traitement dudit modèle de peau reconstituée de l'étape a) par un stress exogène.

Par « stress exogène », on entend tout facteur extérieur affectant l'intégrité de la peau et pouvant entraîner, le cas échéant, une diminution progressive de l'efficacité des fonctions de celle-ci. Les stress exogènes comprennent par exemple les agents polluants, ainsi que les radiations ionisantes et non ionisantes. Plus particulièrement, un stress exogène au sens de la présente divulgation est une substance chimique dont l'application topique conduit à une altération de la structure et/ou de la fonction de la peau. Ces agents sont couramment utilisés dans des études cliniques visant à caractériser la peau sensible (Richters et al., Skin Pharmacol Pysiol, 2015, 28 : 75-83). En particulier, l'utilisation de ces agents permet de définir si une personne possède une peau sensible ou non. De telles substances chimiques incluent notamment des agents tels que l'acide lactique, le sodium lauryl sulfate (SDS), la capsaïcine, le menthol, l'acide benzoïque, l'acide transcinnamique, l'octane, le cumène, le nicotinate de méthyle et le chlorure d'acétyl-b-méthylcholine (vasodilatateurs), l'éthanol, des allergènes, des facteurs d'occlusion cutanée, la cocamidopropyl bétaïne, le chlorure de benzalkonium (tensioactifs) et le baume du Pérou. N'importe lequel de ces agents peut être utilisé pour générer le modèle de peau sensible reconstituée décrit.

Les inventeurs ont plus particulièrement montré que l'application d'acide lactique permet d'obtenir un modèle de peau reconstituée qui reproduit les particularités de la peau sensible. Notamment, l'application d'acide lactique entraine une expression plus importante de marqueurs de l'inflammation sans compromettre la structure histologique de la peau, ni affecter la viabilité cellulaire cutanée. Le stress exogène ici décrit sera donc préférentiellement une application d'acide lactique.

Selon un aspect particulier, le modèle divulgué est susceptible d'être obtenu par un procédé comprenant des étapes de :

a) obtention d'un modèle de peau reconstituée à partir d'un échantillon cutané d'un sujet ; et
b) mise en contact dudit modèle de peau reconstituée de l'étape a) avec de l'acide lactique.

Selon un aspect préféré, le modèle divulgué est obtenu par un procédé comprenant des étapes de :

a) obtention d'un modèle de peau reconstituée à partir d'un échantillon cutané d'un sujet ; et
b) mise en contact dudit modèle de peau reconstituée de l'étape a) avec de l'acide lactique.

L'acide lactique pourra être utilisé à toute concentration permettant de maintenir la structure générale de la peau tout en induisant l'expression des marqueurs de l'inflammation. De façon préférée, la concentration d'acide lactique utilisée est comprise entre 0,05 % et 5 %, préférentiellement entre 0,1 % et 2,5 %, plus préférentiellement entre 0,25 % et 1,25 %, notamment entre 0,4 % et 0,8 %. L'acide lactique pourra être utilisé en solution, à toute concentration permettant de maintenir la structure générale de la peau tout en induisant l'expression des marqueurs de l'inflammation. De façon préférée, la concentration en acide lactique dans la solution utilisée sera comprise entre 0,05 % et 5 %, en poids d'acide lactique/volume de solution. De façon plus préférée, l'acide lactique sera utilisé à une concentration comprise entre 0,1 % et 2,5 %, de façon encore plus préférée, à une concentration comprise entre 0,25 % et 1,25 %, de façon encore plus préférée, à une concentration comprise entre 0,4 % et 0,8 %. Selon l'aspect le plus préféré, l'acide lactique est utilisé à une concentration de 0,6 %, en poids d'acide lactique/volume de solution.

Selon un mode de réalisation de l'invention, le modèle de peau sensible reconstituée est caractérisé en ce que la concentration d'acide lactique utilisée est comprise entre 0,1 % et 2,5 %, préférentiellement entre 0,25 % et 1,25 %, plus préférentiellement entre 0,4 % et 0,8 %.

Selon un autre mode de réalisation, le modèle de peau sensible reconstituée selon l'invention est caractérisé en ce que la concentration d'acide lactique utilisée est de 0,6 %.

Préférablement, ledit sujet est un adulte. Dans ce cas, le modèle de peau divulgué est un modèle de peau d'adulte. Selon un aspect, ledit sujet est un enfant. Dans ce cas, le modèle de peau divulgué est un modèle de peau d'enfant.

Le modèle de peau sensible reconstituée ainsi obtenu est particulièrement avantageux car il reproduit fidèlement les particularités de la peau sensible telles qu'elles ressortent de l'étude clinique conduite par les inventeurs.

Par « peau sensible », on entend ici une condition cutanée neurosensorielle se traduisant par des signes cliniques disparates, dont l'irritation, l'érythème ou la sécheresse cutanée. Plus précisément, la peau sensible est un syndrome défini par la survenue de sensations désagréables, comme par exemple, des piqûres, des brûlures, des douleurs, du prurit ou encore des picotements, en réponse à des stimuli qui ne devraient normalement pas causer de telles sensations ; en outre, la peau sensible se caractérise par l'absence de lien entre lesdites sensations et une pathologie cutanée quelconque (Misery et al., Acta Derm Venereol 2017, 97 : 4-6).

La peau sensible est avantageusement caractérisée par l'expression d'au moins un marqueur biologique.

Dans un aspect préféré, le modèle divulgué exprime au moins un marqueur biologique.

Par exemple, le modèle divulgué est susceptible d'être obtenu par un procédé comprenant des étapes de :

a) obtention d'un modèle de peau reconstituée à partir d'un échantillon cutané d'un sujet ;
b) mise en contact dudit modèle de peau reconstituée de l'étape a) avec de l'acide lactique ; et
c) mesure du niveau d'expression d'au moins un marqueur biologique.

Avantageusement, ledit modèle de peau sensible reconstituée est obtenu par le procédé de préparation d'un modèle décrit ci-après.

Par « marqueur biologique », on entend au sens de la présente demande une caractéristique qui est objectivement mesurée et évaluée comme indicateur de processus biologiques normaux, de processus pathogéniques, ou de réponses pharmacologiques à une intervention thérapeutique. Un marqueur biologique désigne donc toute une gamme de substances et de paramètre divers. Par exemple, un marqueur biologique peut être une substance dont la détection indique un état pathologique particulier (par exemple la présence de la protéine C réactive en tant que marqueur d'une infection), ou au contraire une substance dont la détection indique un état physiologique spécifique. Le marqueur biologique selon la divulgation est préférentiellement un gène, les produits d'un gène tels que ses transcrits et les peptides issus de ses transcrits, un lipide, un sucre ou un métabolite.

L'homme du métier cherchant à déterminer à quelle classe appartient un marqueur biologique pourra facilement consulter la littérature scientifique pertinente ou se rapporter à des bases de données publiques telles que, par exemple, celles regroupées sur le site internet du National Center for Biotechnology Information (http://www.ncbi.nlm.nih.gov/guide/).

Les inventeurs ont particulièrement sélectionné des marqueurs dont le niveau d'expression présente une variation marquée entre une peau sensible et une peau normale. Les marqueurs sélectionnés présentent donc un intérêt particulier dans le cadre de la présente méthode, leur expression étant mesurée dans un modèle de peau reproduisant fidèlement les caractéristiques de la peau sensible.

Les inventeurs ont en particulier montré que les marqueurs de l'inflammation sont particulièrement exprimés dans la peau sensible. L'inflammation est une réaction normale de défense de l'organisme, mais elle peut contribuer à la diminution de l'intégrité de la peau. En outre, les inventeurs ont montré que l'expression des marqueurs de la barrière est diminuée par rapport à la peau normale. Enfin, les marqueurs de l'immunité, dont ceux de l'immunité innée et de la défense sont eux aussi affectés et avec eux la capacité de la peau à réagir aux agressions extérieures.

Le marqueur biologique décrit ici est donc avantageusement un marqueur sélectionné dans le groupe des marqueurs de l'inflammation, des marqueurs de la fonction barrière et des marqueurs de l'immunité innée et de défense.

Plus particulièrement, dans le modèle de peau sensible reconstituée divulgué :

a) l'expression du marqueur biologique choisi dans le groupe des marqueurs de l'inflammation est plus élevée que dans la peau normale, et/ou

b) l'expression du marqueur biologique choisi dans le groupe des marqueurs de la barrière est moins élevée que dans la peau normale ; et/ou

c) l'expression du marqueur biologique choisi dans le groupe des marqueurs de l'immunité innée et de défense est moins élevée que dans la peau normale.

Selon un aspect particulier, le modèle est obtenu par un procédé dans lequel l'expression d'au moins un marqueur biologique sélectionné dans le groupe des marqueurs de l'inflammation, des marqueurs de la fonction barrière et des marqueurs de l'immunité innée et de défense, est mesurée.

Par ailleurs, les inventeurs ont montré que les marqueurs de l'inflammation sont particulièrement exprimés dans la peau sensible. L'inflammation est une réaction normale de défense de l'organisme, mais elle peut contribuer à la diminution de l'intégrité de la peau. En outre, les inventeurs ont montré que dans le même temps, l'expression des marqueurs de la barrière est diminuée par rapport à la peau normale. Enfin, les marqueurs de défense et de l'immunité innée sont eux aussi affectés et avec eux la capacité de la peau à réagir aux agressions extérieures.

Par « marqueurs de l'inflammation cutanée », on entend ici les marqueurs dont la variation d'expression corrèle avec l'inflammation cutanée. On entend par « inflammation » l'ensemble des mécanismes réactionnels de défense par lesquels l'organisme reconnaît, détruit et élimine toutes les substances qui lui sont étrangères. « L'inflammation cutanée » correspond plus particulièrement à une réaction du système immunitaire en réaction à une agression sur la peau, telle qu'une agression de l'environnement, occasionnant ou non une plaie, voire un dommage vasculaire le cas échéant. L'inflammation cutanée peut se manifester par un érythème, caractérisé par une rougeur associée à une vasodilatation locale, un oedème, caractérisée par un gonflement, et une sensation de chaleur. En outre, l'inflammation cutanée s'accompagne d'une variation de niveau d'expression ou de concentration de marqueur géniques, protéiques ou lipidiques bien connus de l'homme du métier, qui pourra se référer par exemple à Vahlquist (Acta Derm Venereol ; 80 : 161 ; 2000).

Le déclenchement et la poursuite de l'inflammation, sa diffusion à partir du foyer initial font appel à des marqueurs inflammatoires qui sont synthétisés localement ou qui sont à l'état de précurseur inactif dans la circulation. Par « marqueur inflammatoire », on entend ici une substance synthétisée et libérée par une cellule de l'organisme agissant sur une cellule du même organisme qui possède un récepteur spécifique de cette substance et intervenant dans les processus de l'inflammation dudit organisme. On peut différencier plusieurs étapes dans la réaction inflammatoire. Ainsi, l'inflammation cutanée telle qu'on l'entend ici fait intervenir au moins deux composantes distinctes, la composante tissulaire et la composante vasculaire.

Par « composante tissulaire » de l'inflammation cutanée, on entend ici le processus cutané local de défense contre une agression. La composante tissulaire de l'inflammation fait intervenir des marqueurs inflammatoires protéiques et des marqueurs inflammatoires lipidiques. Par « marqueurs protéiques », ou « marqueurs inflammatoires protéiques », ou encore « marqueurs protéiques de la composante tissulaire de l'inflammation », on entend ici les marqueurs inflammatoires de nature peptidique ou protéique, tels que les cytokines IL-1, IL-2, IL-6, IL8 et TNFα, le système du complément, ou les protéines impliquées dans la coagulation, le cas échéant. Par « marqueur lipidiques », ou « marqueurs inflammatoires lipidiques », ou encore « marqueurs lipidiques de la composante tissulaire de l'inflammation », on entend ici les marqueurs inflammatoires de nature lipidique, notamment les prostaglandines et les leucotriènes qui sont tous deux synthétisés à partir de l'acide arachidonique, ainsi que l'activation des enzymes responsables de cette production (Shimizu, Annu Rev Pharmacol Toxicol., 49 : 123-150, 2009). Les marqueurs inflammatoires protéiques et lipidiques ainsi produits vont induire une cascade de réactions au sein de la peau impliquant d'autres cellules de l'inflammation, en particulier, des cellules immunitaires et vasculaires. Le résultat clinique se traduit notamment par des rougeurs, voire un oedème.

Durant la réaction inflammatoire, les vaisseaux sanguins et lymphatiques qui se trouvent dans la peau subissent d'abord une dilatation. Cette phase est suivie d'une augmentation de la perméabilité, entraînant un mouvement de l'eau et des sels du vaisseau sanguin ou lymphatique vers le tissu et résultant dans la formation d'un oedème. Par « composante vasculaire », on entend ici ce processus de vasodilatation suivie par un accroissement de la perméabilité vasculaire. La composante vasculaire de l'inflammation comprend notamment les différentes étapes aboutissant à la migration extravasculaire de différents types de leucocytes tels que lymphocytes T CD4$^+$, CD8$^+$, CD4$^-$ CD8$^-$, NK et B, monocytes, polynucléaires neutrophiles, éosinophiles ou basophiles.

Le marqueur de l'inflammation cutanée décrit ici est de préférence choisi parmi les marqueurs protéiques de la composante tissulaire de l'inflammation, les marqueurs lipidiques de la composante tissulaire de l'inflammation et les marqueurs de la composante vasculaire de l'inflammation.

Les marqueurs protéiques de la composante tissulaire de l'inflammation sont bien connus de l'homme du métier. Ils comprennent notamment des cytokines telles que TNFα ou les interleukines, y compris IL-1, IL-1RA, IL-2, IL-6, IL-8, et des métalloprotéases matricielles telles que MMP1a et MMP3. Préférentiellement, le marqueur protéique de la composante tissulaire de l'inflammation est choisi dans le groupe constitué par les interleukines, de préférence IL1α, IL1RA et IL8.

L'interleukine IL1α humaine possède une séquence protéique représentée par la séquence de référence NCBI : NP_000566. Cette protéine est codée par le gène IL1A humain (référence NCBI : Gene ID: 3552), dont la séquence correspond à la référence NCBI : NM_000575. L'interleukine IL1α est le marqueur inflammatoire primaire de la cascade inflammatoire. Cette cytokine sécrétée par de nombreuses cellules en réponse à des stress inflammatoires. Elle est notamment exprimée constitutivement par les kératinocytes, dans le cytoplasme desquels elle est stockée. Elle peut être relarguée passivement en réponse à un stress aigu, tell qu'une altération de la membrane. Cependant, un stress plus modéré peut provoquer en outre une augmentation de la synthèse de cette cytokine.

La protéine IL1RA (IL1 receptor antagonist) est une protéine appartenant à la même famille que IL1α. Sa séquence protéique est représentée par la séquence de référence NCBI : NP_000568. Cette protéine est codée par le gène IL1 RN humain (référence NCBI : Gene ID: 3557), dont la séquence correspond à la référence NCBI : NM_000568. La protéine IL1 RA est exprimée par les cellules épithéliales. Elle est notamment induite en réponse à une irritation, secondairement à IL1α. L'équilibre du rapport IL1RA/IL1α est en particulier important pour maintenir l'homéostasie cutanée. En effet, IL1RA se lie au récepteur de IL1α sans l'activer, atténuant ainsi la réponse induite par IL1α.

La protéine IL8 est une cytokine qui est absente de la peau saine, mais qui est induite précocement dans l'inflammation cutanée. Elle est produite par synthèse de novo dans les kératinocytes stimulés par IL1α ou TNF. De ce fait, c'est un marqueur prédictif d'une réponse biologique, même en l'absence de signes visibles d'irritation cutanée. La séquence protéique de l'interleukine IL-8 humaine correspond à la séquence de référence NCBI : NP_000575. Cette

7

protéine est codée par le gène IL8 humain (référence NCBI : Gene ID: 3576). La séquence de celui-ci est accessible sous la référence NCBI : NM_000584.

Comme expliqué plus haut, le système immunitaire cutané est régulé par des marqueurs inflammatoires tels que les lipides bioactifs qui peuvent déclencher une réponse immunitaire rapide avec une inflammation contrôlée, suivie d'une résolution efficace. Les marqueurs lipidiques représentent l'ensemble de ces marqueurs inflammatoires lipidiques, ainsi que les enzymes affectant leur métabolisme. Ils comprennent des acides gras, dont les acides gras polyinsaturés, notamment les $\omega$-6 et les $\omega$-9, les leucotriènes, les prostaglandines, ainsi que les protéines impliquées dans le métabolisme de ces lipides, comme la phospholipase PLA2G2F, la synthase PTGS2 ou la Glutathione S-Transférase MGST1.

Le marqueur inflammatoire lipidique est choisi avantageusement parmi les acides gras polyinsaturés, notamment les $\omega$-6 et les $\omega$-9, les prostaglandines, dont en particulier la prostaglandine E2, PLA2G2F, et MGST1.

Les acides gras polyinsaturés participent à la transmission du signal inflammatoire. Les acides gras polyinsaturés $\omega$-6, et accessoirement les $\omega$-9, ont ainsi un rôle pro-inflammatoire. L'acide arachidonique est ainsi au coeur de la cascade de l'inflammation lipidique. Cet acide gras 20:4 ($\omega$-6) est libéré à partir des phospholipides membranaires des cellules inflammatoires sous l'action des phospholipases A2. Son métabolisme, notamment par la formation de plusieurs de ses dérivés oxygénés, est associé à des conditions pro-inflammatoires. Parmi les autres acides gras polyinsaturés qui peuvent être utilisés comme marqueurs de l'inflammation lipidique, on peut aussi citer l'acide linoléique (18:2, $\omega$-6) et l'acide oléique (18:1, $\omega$-9).

La prostaglandine E2 (PGE2), est un dérivé bien connu de l'acide arachidonique obtenu par l'action de la cyclo-oxygénase. Il existe deux isoformes de cyclo-oxygénases (COX) : la cyclo-oxygénase 1 qui est constitutive dans les tissus et la cyclo-oxygénase 2 qui est induite par les phénomènes inflammatoires. Une stimulation pro-inflammatoire (traumatisme, cytokines...) conduit ainsi à la synthèse de PGE2 qui est responsable d'une vasodilatation (générant rougeur et oedème), d'une sensibilisation des nocicepteurs à la bradykinine et l'histamine (responsables de la douleur) et de la fièvre (avec les cytokines IL1 et IL6).

Les marqueurs de la composante vasculaire de l'inflammation correspondent aux molécules qui sont impliquées dans les processus de vasodilatation et d'accroissement de la perméabilité vasculaire caractéristiques de l'inflammation vasculaire. Ils comprennent notamment des protéines telles que VEGF-A, FGF2, THBS1, VIPR1, ADCYAP1. Le marqueur de l'inflammation vasculaire est avantageusement le facteur de croissance vasculaire (Vascular Endothelial Growth factor A ; ci-après VEGF-A). Cette protéine est produite par le kératinocyte en réponse à une agression. Elle stimule la prolifération des cellules endothéliales de par son activité angiogénique, ainsi que la perméabilité vasculaire. De fait, la surexpression de VEGF-A conduit à l'extrusion de sang.

Selon un mode de réalisation particulier, le modèle de peau sensible reconstituée selon l'invention est caractérisé en ce que lesdits marqueurs de l'inflammation sont choisis parmi IL1$\alpha$, ILR1A, IL8, les acides gras polyinsaturés $\omega$-6, les acides gras polyinsaturés $\omega$-9, la prostaglandine E2, PLA2G2F, MGST1 et VEGF-A.

Les présents inventeurs ont par ailleurs montré que l'expression des marqueurs de la fonction barrière est réduite dans la peau sensible.

Les « marqueurs de la barrière » selon la présente divulgation comprennent les marqueurs qui sont spécifiquement exprimés dans les couches de l'épiderme les plus externes et qui participent à la fonction barrière.

Comme il est bien connu de l'homme du métier, la peau a pour fonction principale d'établir une barrière de protection contre les atteintes de l'environnement tout en permettant certains échanges entre le milieu intérieur et le milieu extérieur. Cette fonction barrière est principalement portée par le *stratum corneum* de l'épiderme.

Le *stratum corneum* est le produit final de la différenciation de l'épiderme, où les kératinocytes sont transformés en cornéocytes ; ces cornéocytes étant enrobés d'une matrice lipidique intercellulaire constituée de céramides, de cholestérol et d'acides gras essentiels. Après extrusion des corps lamellaires, les précurseurs des lipides sont métabolisés en lipides matures. Dans le cas des précurseurs des céramides, ce sont les enzymes sphingomyélinase acide et beta glucocérébrosidase qui les transforment en céramides 2 et 5 (pour la sphingomyélinase) et en céramides 1, 3, 4, 6, 7, 8 et 9 (pour la glucocérébrosidase). Les cornéocytes sont entourés d'une enveloppe cornée protéique composée de nombreuses protéines comme l'involucrine, la loricrine, les small proline-rich protéines et la scielline notamment, solidement liées sous l'action des transglutaminases. Cependant, sous le *stratum corneum,* les jonctions serrées constituent une seconde ligne de fonction barrière. Ces jonctions constituent au niveau du *stratum granulosum* une barrière de diffusion paracellulaire sélective prévenant de la pénétration de molécules délétères. Les jonctions serrées sont composées de différentes protéines transmembranaires comme notamment les claudines, l'occludine et ZO1.

Les fonctions de barrière portées par le *stratum corneum* et les jonctions serrées sont étroitement liées. En effet, l'altération de l'une ou l'autre peut influencer la formation de l'autre. Préférentiellement, les marqueurs de la fonction barrière selon la présente divulgation sont des marqueurs exprimés dans le *stratum corneum* ou des marqueurs exprimés dans les jonctions serrées du *stratum granulosum.* Ces marqueurs comprennent notamment les claudines, dont la claudine-1 (CLDN1), les transglutaminases, comme par exemple la transglutaminase 1 (TGM1), les kératines, notamment la kératine 1 (KRT1) et la kératine 10 (KRT10), la peptidyl arginine deiminase type 1 humaine (PAD1), la caspase 14 (CASP14), l'aquaporin 3 (AQP3), la loricine (LOR), la scielline (SCEL), la protéine BARX Homeobox 2 (BARX2), la desmogleine-1 (DSG1), la filaggrine (FLG), l'involucrine (IVL), la sphingomyélinase ou sphingomyéline diestérase (SMPD), la cornéodesmosine (CSDN), etc.

Par exemple, ledit marqueur de la barrière épidermique est l'involucrine (IVL). L'involucrine, dont la séquence peptidique est la séquence de référence NCBI : NP_005538.2, est exprimée dans les couches épineuses-granuleuses. C'est le premier précurseur de l'enveloppe cornée qui représente 5 à 15 % de l'enveloppe cornée, et sert de lien également avec l'enveloppe cornée lipidique. Elle est codée par le gène IVL (Gene ID: 3713), dont la séquence a pour référence NCBI : NM_005547.2.

Les inventeurs ont également montré que la quantité de lipides de la peau était diminué dans un modèle de peau sensible. Dans la couche cornée, les lipides sont disposés en plan lamellaires dans les espaces entre les cornéocytes, formant ainsi un ciment qui participe à la protection de la peau contre les agressions extérieures et au maintien d'un bon niveau d'eau intra-épidermique. Ces lipides sont des phospholipides, cholestérol et glucosylcéramides, qui sont modifiés dans les espaces intercornéocytaires, par des enzymes spécialisées, en céramides, cholestérol, sulfate de cholestérol et acides gras libres. (Jungerstend et al., Contact Dermitis, 58(5) : 255-262, 2008). On retrouve également des céramides, des stérols et des acides gras libres dans le sébum, entre autres composants. Enfin, la surface de la peau est recouverte d'un film protecteur qui permet à la peau de maintenir son hydratation et de se protéger contre les agressions extérieures. Ce film cutané de surface comprend, entre autres, un film hydrolipidique, lequel est constitué essentiellement de sueur, d'eau, de sébum et autres lipides. Ceux-ci comprennent des céramides, des triglycérides et des acides gras, en proportions à peu près égales.

Par exemple, le marqueur de la barrière selon la présente divulgation est donc un marqueur lipidique. Par « lipides », on entend ici toute molécule naturelle liposoluble (c'est-à-dire lipophile). Les lipides sont un groupe hétérogène de composés possédant de nombreuses fonctions biologiques essentielles. Les lipides peuvent être définis plus particulièrement comme des petites molécules hydrophobes ou amphiphiles, qui proviennent entièrement ou en partie de groupes cétoacyl ou isoprènes. Pour un aperçu d'ensemble de toutes les classes de lipides, on se reportera avantageusement à « Lipid Metabolites and Pathways Strategy (LIPID MAPS) classification system » (National Institute of General Medical Sciences, Bethesda, MD). En particulier, le marqueur lipidique est choisi parmi les céramides, les phospholipides, les glucosylcéramides, les stérols, les triglycérides et les acides gras libres. De façon encore plus préférée, le marqueur lipidique est choisi parmi les céramides.Un « céramide » selon la divulgation est un lipide issu de la réaction d'amidification de la sphingosine et d'un acide gras. Un céramide est donc constitué d'une base sphingosine ou phytoshingosine liée par une liaison amide à des acides $\alpha$-hydroxy, $\omega$-hydroxy ou non hydroxy possédant des longueurs de chaîne hydrophobe variables. Dans le *stratum corneum* humain, 9 classes de céramides, dénommés CER 1 à 9, ont été identifiées (voir par exemple Dayan, Cosm & Toil, 121(1) : 37-44, 2006 ; Jungerstend et al., Contact Dermitis, 58(5) : 255-262, 2008 ; Farwick et al., Cosm & Toil, 124(2) : 63-72 ; Masukawa et al., J Lipid Res., 50(8) : 1708-1719, 2009). Le céramide selon la divulgation est choisi plus préférentiellement dans le groupe constitué par lesdits céramides CER1 à 9.

D'autre part, les présents inventeurs ont montré que les NMF étaient affectés dans la peau sensible. Le facteur naturel d'hydratation, aussi appelé NMF (Natural Moisturizing Factor) est issu de la protéolyse de la filaggrine selon une cascade de réactions impliquant des enzymes dont notamment la caspase 14 et la peptidylarginine deiminase (PAD1). Le NMF est un mélange de substances hygroscopiques ayant la propriété de retenir l'eau (Fluhr et al., Exp Dermatol., 19(6) : 483-492, 2010). Parmi celles-ci, le sel de sodium de l'acide pyrrolidone carboxylique ou PCA Na (provenant de la cyclisation de l'acide glutamique libéré par la décomposition de la profilaggrine) et les lactates sont les substances les plus hygroscopiques. Le NMF comprend en outre des acides aminés libres (sérine, citrulline...), des citrates et des formiates, de l'urée, des ions, de l'azote, de l'acide urique, de la glycosamine, de la créatinine, des phosphates, ainsi que des composés non encore identifiés. La quantité de NMF peut être mesurée par toutes les méthodes connues de l'homme du métier, notamment par microspectroscopie de Raman. Selon un autre aspect préféré, le marqueur de la barrière est donc le NMF.

Selon un mode de réalisation particulier, le marqueur de la barrière selon l'invention est donc préférablement l'involucrine, un céramide choisi dans le groupe des céramides CER 1 à 9 ou le NMF.

Les présents inventeurs ont également montré que l'expression des marqueurs de l'immunité est réduite dans la peau sensible.

Par « marqueur de l'immunité », on entend ici tous les marqueurs qui servent à déterminer l'identité de l'organisme et à défendre celui-ci contre l'extérieur. Ces marqueurs servent de première ligne de défense contre les infections bactériennes. Ils comprennent des protéines comme les défensines, les beta défensines, notamment les beta défensines 1 et 2 (BD-1 et BD-2), les récepteurs de type Toll, comme par exemple Toll-like receptor 2 et la protéine S100A7. Un marqueur de l'immunité est de préférence la bêta-défensine 2, la protéine S100A7 ou le Toll-like receptor 2.

La bêta-défensine 2 (BD-2) qui est aussi appelée skin-antimicrobial peptide 1 (SAP1) est un peptide codé par le gène DEFB4 (référence NCBI : Gene ID: 1673). La séquence de la bêta-défensine 2 humaine est disponible sous le numéro d'accession NP_004933., tandis que celle du gène DEFB4 l'est sous le numéro NM_004942.

La protéine S100A7, pour S100 calcium binding protein A7 (séquence NP_002954), est une protéine de la famille des protéines S100, lesquelles sont des protéines qui lient le calcium par leurs motifs *EF hands.* Elle est codée par le gène S100A7 (référence NCBI : Gene ID: 1673) de séquence NM_002963.

La protéine Toll-like receptor 2 ou TLR2 (séquence NP_003255.2) est un récepteur membranaire, qui est exprimé sur la surface de certaines cellules. Elle reconnaît les substances étrangères, notamment les lipoprotéines bactériennes. L'activation du TLR2 conduit à la synthèse de cytokines, notamment l'interleukine 6. TLR2 est codé par le gène TLR2 (référence NCBI : Gene ID: 6278) de séquence : NM_003264).

Selon un mode de réalisation préféré, le modèle de peau sensible reconstituée de l'invention est caractérisé en ce que lesdits marqueurs de défense sont choisis parmi la bêta-défensine 2, la protéine S100A7 et le Toll-like receptor 2.

Il sera par ailleurs évident à l'homme du métier que le modèle de peau sensible reproduira d'autant plus fidèlement la situation existant in vivo qu'il exprimera un grand nombre de marqueurs de types différents parmi les marqueurs ci-dessus. Selon un aspect particulier, le modèle de peau décrit est obtenu par un procédé comprenant une étape c) de mesure du niveau d'expression d'une combinaison de marqueurs biologiques. Ladite combinaison selon la divulgation comprend ou consiste en :

- au moins un marqueur de l'inflammation cutanée et au moins un marqueur de la barrière tels que définis plus haut ; ou
- au moins un marqueur de l'inflammation cutanée et au moins un marqueur de l'immunité, tels que définis plus haut ; ou
- au moins un marqueur de la barrière et au moins un marqueur de l'immunité, tels que définis plus haut.

Dans un autre aspect, ladite combinaison comprend au moins un marqueur de l'inflammation cutanée et au moins un marqueur de la barrière et au moins un marqueur de l'immunité, tels que définis plus haut.

L'utilisation de combinaisons de marqueurs comprenant au moins un marqueur de chacun des différents types indiqués plus haut est particulièrement avantageuse.

Pour chacun de ces marqueurs, le terme « niveau d'expression » se réfère à la concentration cellulaire dudit marqueur. Ainsi le niveau d'expression de la prostaglandine E2, d'un céramide ou d'un acide gras polyinsaturé $\omega$-6 ou $\omega$-9 correspond à la concentration dudit lipide dans la cellule. Si le marqueur est un gène, le « niveau d'expression » au sens de la description correspond à la concentration cellulaire d'au moins un des produits du gène dudit marqueur. Plus précisément, le niveau d'expression dudit marqueur biologique correspond à la quantité ou à la concentration cellulaire du transcrit dudit gène ou de la protéine issue dudit transcrit. Par exemple, le niveau d'expression dudit marqueur biologique correspond à la quantité ou à la concentration cellulaire du transcrit dudit gène. Selon un autre aspect, le niveau d'expression dudit marqueur biologique correspond à la quantité ou à la concentration cellulaire de la protéine issue dudit transcrit.

Par la « mesure du niveau d'expression d'une combinaison de marqueurs biologiques », on entend au sens de la présente demande la mesure du niveau d'expression de chacun des marqueurs de la combinaison. L'expression d'un gène peut être mesurée par exemple au niveau nucléotidique, en mesurant la quantité de transcrits dudit gène, et peut aussi être mesurée par exemple au niveau peptidique, en mesurant par exemple la quantité des protéines issus desdits transcrits. Ainsi, par « mesure du niveau d'expression dudit gène » on entend ici la mesure de la quantité ou de la concentration cellulaire de produit du gène sous sa forme peptidique ou sous sa forme nucléotidique.

De façon générale, l'expression du marqueur biologique selon la divulgation sera détectée in vitro à partir du modèle de peau reconstituée.

Dans un aspect particulier, le procédé divulgué peut comprendre une ou plusieurs étapes intermédiaires entre l'obtention du modèle de peau reconstituée et la mesure de l'expression du marqueur biologique, lesdites étapes correspondant à l'extraction à partir dudit modèle de peau reconstituée d'un échantillon lipidique, d'un échantillon de NMF, d'un échantillon d'ARNm (ou de l'ADNc correspondant) ou d'un échantillon de protéine. Celui-ci peut ensuite être directement utilisé pour mesurer l'expression du marqueur. La préparation ou l'extraction d'ARNm (ainsi que la rétrotranscription de celui-ci en ADNc), de protéines, de lipides ou de NMF à partir d'un échantillon de cellules de peau ne sont que des procédures de routine bien connues de l'homme du métier.

En ce qui concerne certains marqueurs lipidiques tels que la prostaglandine E, il peut même ne pas être nécessaire de préparer un échantillon lipidique. En effet, ce marqueur est sécrété dans le milieu de culture. Il est alors aisé pour l'homme du métier de doser la prostaglandine E2 à partir dudit milieu de culture. Plusieurs méthodes pour doser et quantifier la prostaglandine E2 ont ainsi été décrites dans l'art, dont en particulier des méthodes ELISA. Une telle méthode est ainsi détaillée dans la partie expérimentale de la présente demande et l'homme du métier pourra se reporter à celle-ci. Il est en outre à noter que des kits sont disponibles commercialement pour le dosage de la prostaglandine E2 (par exemple, chez CissBio Assays ou chez Pierce).

Une fois qu'un échantillon d'ARNm (ou d'ADNc correspondant) ou de protéine est obtenu, l'expression du marqueur, au niveau soit des ARNm (c'est-à-dire dans l'ensemble des ARNm ou des ADNc présents dans l'échantillon), soit des protéines (c'est-à-dire dans l'ensemble des protéines présentes dans l'échantillon), peut être mesurée. La méthode utilisée pour ce faire dépend alors du type de transformation (ARNm, ADNc ou protéine) et du type d'échantillon disponible.

Quand l'expression du marqueur est mesurée au niveau de l'ARNm (ou d'ADNc correspondant), n'importe quelle technologie habituellement utilisée par l'homme du métier peut être mise en oeuvre. Ces technologies d'analyse du niveau d'expression des gènes, comme par exemple l'analyse du transcriptome, incluent des méthodes bien connues telles que la PCR (Polymerase Chain Reaction, si on part d'ADN), la RT-PCR (Reverse Transcription-PCR, si on part d'ARN) ou la RT-PCR quantitative ou encore les puces d'acides nucléiques (dont les puces à ADN et les puces à oligonucléotides) pour un plus haut débit.

Par « puces d'acides nucléiques », on entend ici plusieurs sondes d'acides nucléiques différentes qui sont attachées à un substrat, lequel peut être une micropuce, une lame de verre, ou une bille de la taille d'une microsphère. La micropuce peut être constituée de polymères, de plastiques, de résines, de polysaccharides, de silice ou d'un matériau à base de silice, de carbone, de métaux, de verre inorganique, ou de nitrocellulose.

Les sondes peuvent être des acides nucléiques tels que les ADNc (« puce à ADNc »), les ARNm (« puce à ARNm ») ou des oligonucléotides (« puce à oligonucléotides »), lesdits oligonucléotides pouvant typiquement avoir une longueur comprise entre environ 25 et 60 nucléotides.

Pour déterminer le profil d'expression d'un gène particulier, un acide nucléique correspondant à tout ou partie dudit gène est marqué, puis mis en contact avec la puce dans des conditions d'hybridation, conduisant à la formation de complexes entre ledit acide nucléique cible marqué et les sondes attachées à la surface de la puce qui sont complémentaires de cet acide nucléique. La présence de complexes hybridés marqués est ensuite détectée.

Ces technologies permettent de suivre le niveau d'expression d'un gène en particulier ou de plusieurs gènes voire même de tous les gènes du génome (full genome ou full transcriptome) dans un échantillon biologique (cellules, tissus...). Ces technologies sont utilisées en routine par l'homme du métier et il n'est donc pas besoin de les détailler ici. Des exemples de mises en oeuvre basées sur l'analyse d'expression génique (puces à ADNc) et sur la PCR quantitative sont décrits dans la section expérimentale.

Alternativement, il est possible d'utiliser toute technologie actuelle ou future permettant de déterminer l'expression des gènes sur la base de la quantité d'ARNm dans l'échantillon. Par exemple, l'homme du métier peut mesurer l'expression d'un gène par hybridation avec une sonde d'acide nucléique marquée, comme par exemple par Northern blot (pour l'ARNm) ou par Southern blot (pour l'ADNc), mais aussi par des techniques telles que la méthode d'analyse sérielle de l'expression des gènes (SAGE) et ses dérivés, tels que LongSAGE, SuperSAGE, DeepSAGE, etc. Il est aussi possible d'utiliser des puces à tissu (aussi connues en tant que TMAs : « tissue microarrays »). Les tests habituellement employés avec les puces à tissu comprennent l'immunohistochimie et l'hybridation fluorescente in situ. Pour l'analyse au niveau de l'ARNm, les puces à tissu peuvent être couplées avec l'hybridation fluorescente in situ. Enfin, il est possible d'utiliser le séquençage massif en parallèle pour obtenir déterminer la quantité d'ARNm dans l'échantillon (RNA-Seq ou « Whole Transcriptome Shotgun Sequencing »). À cet effet, plusieurs méthodes de séquençage massif en parallèle sont disponibles. De telles méthodes sont décrites dans, par exemple, US 4,882,127 ; US 4,849,077 ; US 7,556,922 ; US 6,723,513 ; WO 03/066896 ; WO 2007/111924 ; US 2008/0020392 ; WO 2006/084132 ; US 2009/0186349 ; US 2009/0181860 ; US 2009/0181385 ; US 2006/0275782 ; EP-B1-1141399 ; Shendure & Ji, Nat Biotechnol., 26(10) : 1135-45, 2008 ; Pihlak et al., Nat Biotechnol., 26(6) : 676- 684, 2008 ; Fuller et al., Nature Biotechnol., 27(11) : 1013-1023, 2009 ; Mardis, Genome Med., 1(4): 40, 2009 ; Metzker, Nature Rev. Genet., 11(1) : 31-46, 2010.

Quand l'expression du marqueur est mesuré au niveau protéique, il est possible d'employer des anticorps spécifiques, en particulier dans des technologies bien connues telles que l'immunoprécipitation, l'immunohistologie, le western blot, le dot blot, l'ELISA ou l'ELISPOT, les puces à protéines, les puces à anticorps, ou les puces à tissu couplées à l'immunohistochimie. Parmi les autres techniques qui peuvent être utilisées sont comprises les techniques de FRET ou de BRET, la cytométrie de flux, les méthodes de microscopie ou d'histochimie, dont notamment les méthodes de microscopie confocale, de microscopie à fluorescence, de microscopie électronique, de microscopie à force atomique, les méthodes basées sur l'utilisation d'une ou plusieurs longueurs d'onde d'excitation et d'une méthode optique adaptée, comme une méthode électrochimique (les techniques voltamétrie et d'ampérométrie), et les méthodes de radiofréquence, comme la spectroscopie résonance multipolaire, confocale et non-confocale, comme la détection de fluorescence, luminescence, chemiluminescence, absorbance, réflectance, transmittance, biréfringence ou index de réfraction (par exemple, par résonance des plasmons de surface, ou « surface plasmon résonance » en anglais, par ellipsométrie, par méthode de miroir résonnant, etc.), l'imagerie par résonance radioisotopique ou magnétique, l'analyse par électrophorèse en gel de polyacrylamide (SDS-PAGE); par électrophorèse bidimensionnelle, par spectrophotométrie, par spectrométrie de masse et spectrométrie de masse en tandem, par chromatographie liquide ou gazeuse couplée à la spectrométrie de masse ou à la spectrométrie de masse en tandem. Toutes ces techniques sont bien connues de l'homme du métier et il n'est pas nécessaire de les détailler ici.

Si le marqueur biologique est un lipide, notamment un céramide ou un acide gras, l'homme du métier pourra utiliser toutes les méthodes à sa disposition pour mesurer la teneur en lipide dans un échantillon de cellules cutanées. Ces méthodes comprennent, entre autres, la chromatographie liquide (HPLC, voir par exemple Sullivan et al., Arch Ophthalmol., 120(12) : 1689-99, 2002), la chromatographie liquide couplée à un détecteur évaporatif à diffraction de la lumière (HPLC-ESD, voir Nordbäck et al., J. High Resolut. Chromatogr., 22 : 483-486, 1999 ; Torres et al., J. Chromatogr. A., 1078 : 28-34, 2005) ; la chromatographie en couche mince (TLC, par exemple Downing et al., J Invest Dermatol., 77(4) : 358-360, 1981; Nordstrom et al., J Invest Dermatol., 87(2) : 260-263, 1986) ; la résonance magnétique nucléaire (NMR, voir par exemple Robosky et al., J Lipid Res., 49(3) : 686-692, 2008) ; la microspectroscopie confocale in vivo de Raman ; la spectrométrie de masse, la chromatographie en phase gazeuse couplée à la spectrométrie de masse (GC-MS, voir O'Neill et al., J Chromatogr Sci., 14(1) : 28-36, 1976) ; la chromatographie en phase gazeuse couplée à un détecteur à ionisation de flamme ; la chromatographie en phase liquide couplée à la spectrométrie de masse (voir par exemple van Smeden et al., J Lipid Res, 52(6):1211-1221, 2011) ; la chromatographie en phase liquide ultra-performante (UPLC, voir Rainville et al., J Proteome Res., 6(2):552-558, 2007 ; Castro-Perez et al., J Proteome Res., 10(9) : 4281-4290, 2011). On peut également analyser l'organisation de ces lipides dans la peau et plus particulièrement dans le *stratum corneum* (ou couche cornée), organisation lamellaire ou latérale, par des techniques par exemple de diffraction aux rayons X (Bouwstra et al., J Invest Dermatol., 97(6): 1005-1012, 1991 ; van Smeden et al., J Lipid Res., 52(6):1211-1221, 1991) ou par spectroscopie infra-rouge à transformée de Fourier (Gorcea et al., Int J Pharm. Nov 10, 2011) ou par une analyse morphométrique en microscopie électronique (Daehnhardt-Pfeiffer et al., Skin Pharmacol Physiol., 25(3): 155-161, 2012) ou par une analyse en microscopie électronique de section vitreuse de peau combinée à une analyse moléculaire (Iwai et al., J Invest Dermatol., Apr 26, 2012).

Le dosage du NMF est une procédure bien connue de l'homme du métier. Il est en particulier possible de doser le NMF par l'utilisation de microspectroscopie confocale in vivo de Raman. C'est une procédure couramment utilisée dans le domaine depuis au moins 15 ans. A titre d'exemple, on citera entre autres les publications de Caspers et al. (J Invest Dermatol., 116(3) : 434-442, 2001), Vyumvuhore et al. (J Biomed Opt., 19(11) : 111603, 2014), ou encore Falcone et al. (Skin Pharmacol Physiol, 28 : 307-317, 2015). Il est aussi possible de doser les NMF par la chromatographie en phase liquide couplée à la spectrométrie de masse. On se référera par exemple à Piraud et al. (Rapid Commun Mass Spectrom, 19(12) :1587-602, 2005), Petritis et al. (Journal of Chromatography A, 833(2): 147-155, 1999), Henriksen et al. (J Am SocMass Spectrom, 16(4): 446-455, 2005) ou encore Yang (Application of biophysics and bioengineering to the assessment of skin barrier function. Thesis (Doctor of Philosophy (PhD)). University of Bath, UK, 2011).

Le modèle de peau sensible selon la divulgation est un modèle de peau reconstituée dans lequel l'expression des marqueurs ci-dessus est augmentée ou diminuée, selon le marqueur, par rapport à la peau normale.

Par « peau normale », on entend ici une peau provenant d'un sujet sain. Afin d'éviter toute ambiguïté, on précisera que la peau sensible n'est pas une peau normale au sens de la présente divulgation.

Le terme « augmenté », tel qu'il est utilisé ici, signifie une plus grande quantité, par exemple, une quantité légèrement supérieure à la quantité d'origine, ou par exemple une quantité en grand excès par rapport à la quantité d'origine, et notamment toutes les quantités dans l'intervalle. En variante, « augmentation » peut faire référence à une quantité ou une activité qui est au moins 1 %, 2 %, 3 %, 4 %, 5 %, 6 %, 7 %, 8 %, 9 %, 10 %, 11 %, 12 %, 13 %, 14 %, 15 %, 16 %, 17 %, 18 %, 19 % ou 20 % de plus que la quantité ou de l'activité pour laquelle la quantité ou de l'activité accrue est comparé, ou encore au moins 30 %, 40 %, 50 %, 60 %, 70 %, 80 %, 90 %, 100 %, 110 %, 120 %, 130 %, 140 %, 150 %, 160 %, 170 %, 180 %, 190 %, 200 %, 250 %, 300 %, 350 %, 400 %, 450 %, 500 %, 550 %, 600 %, 650 %, 700 %, 750 %, 800 %, 900 %, 950 %, 1 000 %, 1 100 %, 1 200 %, 1 300 %, 1 400 %, 1 500 %, 1 600 %, 1 700 %, 1 800 %, 1 900 % ou 2 000 % de plus que la quantité ou de l'activité pour laquelle la quantité ou de l'activité accrue est comparé. Les termes « augmenté », « plus grand que », et « accru » sont utilisés ici de manière interchangeable.

Le terme « diminué », tel qu'il est utilisé ici, signifie une moins grande quantité, par exemple, une quantité légèrement inférieure à la quantité d'origine, ou par exemple une quantité fortement réduite par rapport à la quantité d'origine, et notamment toutes les quantités dans l'intervalle. En variante, « diminution » peut faire référence à une quantité ou une activité qui est au moins 1 %, 2 %, 3 %, 4 %, 5 %, 6 %, 7 %, 8 %, 9 %, 10 %, 11 %, 12 %, 13 %, 14 %, 15 %, 16 %, 17 %, 18 %, 19 % ou 20 % de moins que la quantité ou de l'activité pour laquelle la quantité ou de l'activité diminuée est comparé, ou encore au moins 30 %, 40 %, 50 %, 60 %, 70 %, 80 %, 90 %, 100 %, 110 %, 120 %, 130 %, 140 %, 150 %, 160 %, 170 %, 180 %, 190 %, 200 %, 250 %, 300 %, 350 %, 400 %, 450 %, 500 %, 550 %, 600 %, 650 %, 700 %, 750 %, 800 %, 900 %, 950 %, 1 000 %, 1 100 %, 1 200 %, 1 300 %, 1 400 %, 1 500 %, 1 600 %, 1 700 %, 1 800 %, 1 900 % ou 2 000 % de moins que la quantité ou de l'activité pour laquelle la quantité ou de l'activité diminuée est comparé. Les termes « diminué », « plus petit que », et « réduit » sont utilisés ici de manière interchangeable.

Afin de déterminer si l'expression d'un marqueur est accrue ou diminuée dans le modèle de peau sensible de la divulgation, le niveau d'expression dudit marqueur pourra être comparé avec un niveau d'expression de référence.

Selon un aspect préféré, le modèle de peau sensible est obtenu par un procédé comprenant en outre une étape d) de comparaison du niveau d'expression du marqueur biologique de l'étape c) avec un niveau d'expression de référence.

Par « un niveau d'expression de référence d'un marqueur biologique », on entend au sens de la présente demande tout niveau d'expression dudit marqueur utilisé à titre de référence. Par exemple, un niveau d'expression de référence peut être obtenu en mesurant le niveau d'expression du marqueur d'intérêt dans un modèle de peau normale. Un tel modèle de peau normale peut, par exemple, correspondre à un modèle de peau reconstituée obtenu à partir d'un échantillon cutané d'un sujet sain. On peut aussi utiliser comme modèle de peau normale, le modèle de peau reconstituée de l'étape a) avant mise en contact avec l'acide lactique. Dans ce cas, l'expression du marqueur biologique est mesurée avant l'étape b) ; le niveau ainsi déterminé est alors le niveau d'expression de référence dudit marqueur biologique.

Dans d'autres exemples, le niveau d'expression de référence d'un marqueur biologique correspond au niveau d'expression dudit marqueur dans le modèle de peau sensible en absence ou en présence d'un traitement particulier. Par exemple, le niveau d'expression de référence d'un marqueur biologique est obtenu en mesurant l'expression dudit marqueur dans le modèle de peau sensible qui n'a pas été mis en contact avec un actif ou une formulation. Dans un autre exemple, l'expression dudit marqueur est mesurée dans le modèle de peau sensible traité avec un actif ou une formulation connus pour être efficaces contre la peau sensible.

L'homme du métier comprendra en outre aisément que la comparaison de l'étape d) est de préférence effectuée entre des mesures de niveaux d'expression obtenus pour des modèles de peau obtenus à partir d'échantillons cutanés de structures histologiques similaires, voire identiques. Par « structures histologiques similaires », on entend au sens de la présente demande que les proportions relatives des types cellulaires compris dans les modèles de peau comparés sont similaires. Ainsi, il est préférable que les proportions relatives des types cellulaires compris dans le modèle de peau de l'étape a) ne diffèrent pas de plus de 5 % des proportions relatives des types cellulaires compris dans le modèle de peau utilisé pour l'obtention du niveau d'expression de référence de l'étape d). Par « proportion relative d'un type cellulaire » on entend au sens de la présente demande le rapport du nombre de cellules correspondant à ce type cellulaire sur le nombre de cellules totales comprises dans le modèle de peau. Ainsi, il est par exemple préférable que la proportion de kératinocytes sur le nombre de cellules totales dans le modèle de peau de l'étape a) ne diffère pas de plus de 5 % de la proportion de kératinocytes sur le nombre de cellules totales dans le modèle de peau utilisé pour l'obtention du niveau d'expression de référence de l'étape d). Par « structures histologiques identiques », on entend au sens de la présente demande que les proportions relatives des types cellulaires compris dans les modèles de peau comparés sont identiques. Au sens de la présente demande, les proportions relatives des types cellulaires compris dans le modèle de peau de l'étape a) sont identiques aux proportions relatives des types cellulaires compris dans le modèle de peau utilisé pour l'obtention du niveau d'expression de référence de l'étape d) lorsqu'elles ne diffèrent pas de plus de 0,1 %. Avantageusement, la proportion de kératinocytes sur le nombre de cellules totales dans le modèle de peau de l'étape a) ne diffère pas de plus de 0,1% de la proportion de kératinocytes sur le nombre de cellules totales dans le modèle de peau utilisé pour l'obtention du niveau d'expression de référence de l'étape d).

L'homme du métier comprendra tout aussi aisément que la comparaison de l'étape d) est de préférence effectuée entre des mesures de niveaux d'expression obtenus pour des modèles de peau qui sont de taille, de volume ou de poids similaires, voire identiques. Ainsi, il est préférable que la taille, le volume, ou le poids du modèle de peau de l'étape a) ne diffère pas de plus de 5 % de la taille, du volume, ou du poids du modèle de peau utilisé pour l'obtention du niveau d'expression de référence de l'étape d). Plus préférentiellement, la taille, et le volume et le poids du modèle de peau de l'étape a) ne diffèrent pas de plus de 5 % de la taille, du volume et du poids du modèle de peau utilisé pour l'obtention du niveau d'expression de référence de l'étape d). Encore plus préférentiellement, la taille, et le volume et le poids du modèle de peau de l'étape a) ne diffèrent pas de plus de 0,1 % de la taille, du volume et du poids du modèle de peau utilisé pour l'obtention du niveau d'expression de référence de l'étape d).

Alternativement, si les modèles de peau diffèrent de plus de 5 % de par la taille, le volume et le poids, l'homme du métier pourra normaliser le niveau obtenu à l'étape c) et le niveau de référence de l'étape d) à l'aide d'un facteur de normalisation.

Ce facteur de normalisation pourra par exemple être un marqueur physique directement accessible tel que la masse de cellules de l'échantillon, ou la masse d'un constituant cellulaire, comme la masse d'ADN cellulaire ou la masse de protéines cellulaires.

Il peut aussi être avantageux d'utiliser comme facteur de normalisation le niveau d'expression d'un gène qui est exprimé au même niveau dans toutes les cellules, ou presque, de l'organisme. En d'autres termes, selon un aspect particulier, on utilise comme facteur de normalisation le niveau d'expression d'un gène de ménage. Selon un autre aspect, le niveau obtenu à l'étape c) et le niveau de référence de l'étape d) sont normalisés en utilisant le niveau d'expression, non pas des gènes de ménage, mais des protéines codées par ceux-ci. Un gène de ménage est un gène exprimé dans tous les types cellulaires, qui code une protéine ayant une fonction de base nécessaire pour la survie de tous les types cellulaires. Une liste de gènes de ménage humains peut être trouvée dans Eisenberg et al. (Trends in Genet, 19 : 362-365, 2003). Les gènes de ménage divulgués ici incluent par exemple les gènes RPS28, GAPDH, B2M, TFRC, YWHAZ, RPLO, 18S, GUSB, UBC, TBP, GAPDH, PPIA, POLR2A, ACTB, PGK1, HPRT1, IPO8 et HMBS.

Au sens de la présente demande, le modèle de peau reconstituée obtenu à partir d'un échantillon cutané peut être tout modèle tissulaire comprenant des cellules de la peau, notamment des kératinocytes, et dans lequel lesdites cellules cutanées ont été obtenues à partir d'un échantillon cutané.

Par « échantillon cutané », on entend ici tout échantillon contenant des cellules de la peau. Les échantillons cutanés selon la demande comprennent donc aussi bien les explants de peau frais obtenus directement du patient, que les cultures de cellules cutanées en suspension, les cultures de cellules cutanées en monocouche, les cultures de cellules cutanées en bicouche et les modèles tissulaires, dont les cultures de peaux reconstruites et les cultures de muqueuses reconstruites. Comme il est souvent difficile de travailler sur des explants frais, il est particulièrement avantageux, dans le cadre de la présente demande, d'utiliser des cultures de cellules cutanées. Avantageusement, les cellules cutanées selon la divulgation comprennent des cellules normales, saines ou pathologiques, ou des cellules issues de lignées. Par exemple, les cellules cutanées mises en culture peuvent être des cellules obtenues à partir d'explant de tissu cutané. Par « explant » ou « explant de peau », on entend ici un prélèvement de cellules ou de tissu cutané, lequel peut être réalisé dans un but chirurgical ou pour effectuer des analyses.

En particulier, un explant peut être obtenu lors d'une exérèse chirurgicale. Par « exérèse », on entend ici une intervention chirurgicale consistant à découper (exciser) une partie plus ou moins large ou profonde de la peau pour en traiter une anomalie ou une excroissance. On procède à une exérèse soit pour retirer une tumeur cancéreuse ou suspecte de l'être, soit pour traiter une anomalie bénigne de la peau qui est gênante, que ce soit pour des raisons fonctionnelles ou esthétiques. Une exérèse au sens de la demande inclut par exemple les échantillons de peau obtenus après chirurgie plastique (plastie mammaire, abdominale, lifting, prélèvement préputial, otoplastie, c'est-à-dire recollement d'oreille, syndactylie ou doigt surnuméraire, etc.).

Un explant peut aussi être obtenu par biopsie. Par « biopsie », on entend ici un prélèvement de cellules ou tissu cutané réalisé à des fins d'analyse. Plusieurs types de procédures de biopsies sont connus et pratiqués dans le domaine. Les types les plus communs comprennent (1) la biopsie incisionnelle, dans laquelle seul un échantillon du tissu est prélevé ; (2) la biopsie excisionnelle (ou biopsie chirurgicale) qui consiste en l'ablation totale d'une masse tumorale, réalisant ainsi un geste thérapeutique et diagnostique, et (3) la biopsie à l'aiguille, dans laquelle un échantillon de tissu est prélevé avec une aiguille, celle-ci pouvant être large ou fine. D'autres types de biopsie existent, comme par exemple le frottis ou le curettage, et sont aussi englobés dans la présente demande.

Alternativement, lesdites cellules cutanées peuvent être obtenues par différentiation de cellules souches (Guenou et al., Lancet, 374(9703) : 1745-1753, 2009 ; Nissan et al., Proc. Natl. Acad. Sci., 108(36) : 14861-14866, 2011 ; Kraehenbuehl et al., Nature Methods, 8 : 731-736, 2011). Selon un aspect, lesdites cellules souches ne sont pas des cellules souches embryonnaires humaines.

Les cellules cutanées selon la demande, qu'elles proviennent d'une biopsie ou qu'elles soient obtenues par différentiation de cellules souches, comprennent au moins un type de cellules habituellement présentes dans l'hypoderme, le derme et/ou l'épiderme. Ces cellules comprennent ainsi, entre autres, des kératinocytes, des mélanocytes, des fibroblastes, des adipocytes, des cellules endothéliales, des mastocytes, des cellules de Langerhans et/ou des cellules de Merkel. De façon préférentielle, les cellules cutanées selon la divulgation comprennent au moins des kératinocytes et/ou des fibroblastes. De façon plus préférentielle, les cellules cutanées selon la divulgation comprennent des kératinocytes et/ou des fibroblastes.

Selon un mode de réalisation particulier de l'invention, le modèle de peau sensible reconstituée est caractérisé en ce que les cellules dudit modèle proviennent d'un explant de tissu cutané ou de cellules souches différenciées en cellules cutanées.

Selon un autre mode de réalisation particulier de l'invention, le modèle de peau sensible reconstituée est caractérisé en ce que ledit modèle comprend au moins des fibroblastes ou des kératinocytes.

De nombreuses méthodes de culture de cellules cutanées sont connues de l'homme du métier. N'importe laquelle de ces méthodes peut être utilisée pour cultiver les cellules cutanées de la demande. Avantageusement, les cellules cutanées sont cultivées et/ou conservées dans des conditions maintenant, au moins partiellement, un métabolisme cellulaire et/ou des fonctions cellulaires. La culture de cellules cutanées selon la demande comprend donc aussi bien les cultures de cellules cutanées en suspension, les cultures de cellules cutanées en monocouche ou les cultures de cellules cutanées en bicouche que des modèles tissulaires, dont les cultures de peaux reconstruites et les cultures de muqueuses reconstruites.

Par exemple, les cultures de cellules cutanées en suspension sont pratiquées en routine dans un très grand nombre de laboratoires et ce, depuis plusieurs dizaines d'années. De même, les cultures de cellules cutanées en monocouche ou en bicouche sont connues et utilisées depuis très longtemps.

Par ailleurs, de nombreux modèles tissulaires, dont en particulier des modèles de peaux reconstruites et des modèles de muqueuses reconstruites (Rosdy et al., In Vitro Toxicol., 10(1) : 39-47, 1997 ; Ponec et al., J Invest Dermatol., 109(3) : 348-355, 1997 ; Ponec et al., Int J Pharm., 203(1-2) : 211-225, 2000 ; Schmalz et al., Eur J Oral Sci., 108(5) : 442-448, 2000 ; Black et al., Tissue Eng, 11(5-6) : 723-733, 2005 ; Dongari-Batgtzoglou et Kashleva, Nat Protoc, 1(4) : 2012-2018, 2006 ; Bechtoille et al., Tissue Eng, 13(11) : 2667-2679, 2007 ; Vrana et al., Invest Ophthalmol Vis Sci, 49(12) : 5325-5331, 2008 ; Kinicoglu et al., Biomaterials, 30(32) : 6418-6425, 2009 ; Auxenfans et al., Eur J Dermatol, 19(2) : 107-113, 2009; Kinicoglu et al., Biomaterials, 32(25) : 5756-5764, 2011 ; Costin et al., Altern Lab Anim, 39(4) : 317-337, 2011 ; Auxenfans et al., J Tissue Eng Regen Med, 6(7) : 512-518, 2012 ; Lequeux et al., Skin Pharmacol Physiol, 25(1) : 47-55, 2012 ; EP 29 678 ; EP 285 471 ; EP 789 074 ; EP 1 451 302 B1 ; EP 1 878 790 B1 ; EP 1 974 718 ; US 2007/0148,771 ; US 2010/0,099,576 ; WO 02/070729 ; WO 2006/063864 ; WO 2006/0,63865 ; WO 2007/064305) sont à la disposition de l'homme du métier.

Selon un mode de réalisation particulier de l'invention, le modèle de peau sensible reconstituée est caractérisé en ce que le modèle de peau reconstituée de l'étape a) est choisi parmi les cultures de cellules cutanées en suspension, les cultures de cellules cutanées en monocouche, les cultures de cellules cutanées en bicouche, les cultures de peaux reconstruites et les cultures de muqueuses reconstruites.

Avantageusement, le modèle tissulaire comprend les modèles de peau reconstruite et les modèles de muqueuse reconstruite. Préférablement, le modèle de peau reconstruite est sélectionné dans le groupe comprenant les modèles de derme, contenant principalement des cellules stromales, et plus particulièrement encore des fibroblastes, les modèles d'épiderme constitué principalement de kératinocytes, les modèles d'hypoderme, les modèles de peau comprenant un derme et un épiderme, et les modèles de peau comprenant un derme, un épiderme et un hypoderme. Les modèles comprenant au moins un derme, forment des tissus de type conjonctifs, tandis que les modèles comprenant au moins un épiderme forment des épithélia stratifiés comprenant les couches caractéristiques du tissu considéré. Par exemple, on peut identifier dans les modèles d'épiderme une couche basale (*stratum basalis*), une couche épineuse (*stratum spinosum*), une couche granuleuse (*stratum granulosum*), et une couche cornée (*stratum corneum*). D'autre part, de façon préférée, le modèle de muqueuse reconstruite selon la demande est un modèle de muqueuse de la bouche, de la gencive, du vagin ou de la cornée.

Avantageusement, ledit modèle est un modèle tissulaire de type conjonctif de matrice de derme comprenant un support matriciel de préférence choisi parmi :

- un support inerte choisi parmi le groupe consistant d'une membrane synthétique semi-perméable, en particulier une membrane de nitrocellulose semi-perméable, une membrane de nylon semi- perméable, une membrane ou une éponge de téflon, une membrane de polycarbonate ou de polyéthylène, polypropylène, polyéthylène téréphtalate (PET) semi-perméable, une membrane inorganique Anopore semi-perméable, d'acétate ou d'ester de cellulose (HATF), une membrane Biopore-CM semi-perméable, une membrane de polyester semi-perméable, une membrane ou un film d'acide polyglycolique.

Dans ce groupe on trouve par exemple les modèles dermiques Skin$^{2™}$ modèle ZK1100 et Dermagraft® et Transcyte® (Advanced Tissue Sciences) ;

- un plastique traité culture cellulaire (formation d'un feuillet dermique : Michel et al., In Vitro Cell. Dev Biol.-Animal, 35 : 318-326, 1999) ;
- un gel ou une membrane à base d'acide hyaluronique (Hyalograft® 3D - Fidia Advanced Biopolymers) et/ou de collagène (comme par exemple un derme équivalent ou des lattices de collagène) et/ou de fibronectine et/ou de fibrine ; dans ce groupe on trouve par exemple le modèle dermique Vitrix® (Organogenesis) ;
- une matrice poreuse, surfacée ou non surfacée (par exemple un derme équivalent), réalisée à partir de collagène pouvant contenir un ou plusieurs glycosaminoglycanes et/ou éventuellement du chitosane (EP 0296078A1, WO 01/911821 et WO 01/92322).

Dans ce groupe on trouve par exemple le modèle dermique Mimederm® (BASF Beauty Care Solutions).

Ces supports matriciels comprennent des cellules stromales, en particulier des fibroblastes.

Avantageusement, ledit modèle de peau est un modèle d'épiderme comprenant un support matriciel de préférence choisi parmi :

- un support inerte choisi parmi le groupe consistant d'une membrane synthétique semi-perméable, en particulier une membrane de nitrocellulose semi-perméable, une membrane de nylon semi- perméable, une membrane ou une éponge de téflon, une membrane de polycarbonate ou de polyéthylène, polypropylène, de polyéthylène téréphtalate (PET) semi-perméable, une membrane inorganique Anopore semi-perméable, d'acétate ou d'ester de cellulose (HATF), une membrane Biopore-CM semi-perméable, une membrane de polyester semi-perméable ;

dans ce groupe on trouve les modèles Epiderme reconstruits (Skinethic®) ainsi que le modèle EpiDerm®, (Mattek Corporation) ;

- un film ou une membrane à base d'acide hyaluronique et/ou de collagène et/ou de fibronectine et/ou de fibrine.

Dans ce groupe on peut citer particulièrement les modèles : Laserskin® (Fidia Advanced Biopolymers), Episkin ® (L'Oréal).

Ces modèles peuvent être ensemencés par des fibroblastes dans la partie dermique.

Ces modèles, dans lesquels peuvent être intégrés ou non des fibroblastes, servent de support pour l'ensemencement des kératinocytes et la reconstitution de l'épiderme. Avantageusement, sont introduites, outre les kératinocytes, des cellules pigmentaires, des cellules immunocompétentes, des cellules nerveuses ; de préférence les cellules immunocompétentes sont des cellules de Langerhans.

Avantageusement, ledit modèle tissulaire est un modèle tissulaire de peau ou de muqueuse reconstruite comprenant un support matriciel dermique ou de chorion de préférence choisi parmi :

- un support inerte choisi parmi le groupe consistant d'une membrane synthétique semi-perméable, en particulier une membrane de nitrocellulose semi-perméable, une membrane de nylon semi- perméable, une membrane ou une éponge de téflon, une membrane de polycarbonate ou de polyéthylène, polypropylène, de polyéthylène téréphtalate (PET) semi-perméable, une membrane inorganique Anopore semi-perméable, d'acétate ou d'ester de cellulose (HATF), une membrane Biopore-CM semi-perméable, une membrane de polyester semi-perméable, ledit support inerte contenant ou non des cellules stromales, en particulier des fibroblastes,
- un gel à base de collagène et/ou acide hyaluronique et/ou fibronectine, et/ou de fibrine comprenant des cellules stromales en particulier des fibroblastes,
- une matrice poreuse, surfacée ou non surfacée, réalisée à partir de collagène pouvant contenir un ou plusieurs glycosaminoglycanes et/ou éventuellement du chitosane, ces matrices poreuses intégrant des cellules stromales, en particulier des fibroblastes,
- un derme desépidermisé ou derme mort, humain ou animal.

Dans ce groupe, on peut citer particulièrement les modèles Mimeskin (BASF Beauty Care Solutions), Epidern-mFT™, EpiAirway™, EpiOccular™ EpiOral™, EpiGingival™, EpiVaginal™ (MatTek corporation), Human Corneal Epithelium (HCE), Human Oral Epithelium (HOE), Human Gingival Epithelium (HGE), Human Vaginal Epithelium (HVE) (Skinethic®), Phenion® Full Thickness Skin Model (Phenion) Apligraf® (Organogenesis), ATS-2000 (CellSystems® Biotechnologie Vertrieb) ainsi que Skin 2TM (ZK1200-1300-2000 Advanced Tissue Science).

Il existe par ailleurs des modèles dédiés à la thérapie tissulaire qui peuvent également être utilisés dans le cadre de la présente demande. On peut citer les modèles Epidex (Modex Thérapeutiques), Epibase® (Laboratoire Genévrier), Epicell™ (Genzyme), Autoderm™ et Transderm™ (Innogenetics).

Le support matriciel est ensuite ensemencé par des kératinocytes pour reconstruire l'épiderme et obtenir finalement une peau reconstruite.

Avantageusement, le modèle de peau utilisé comprend un modèle dans lequel a été incorporé au moins un type cellulaire complémentaire, comme des cellules endothéliales (CE) et/ou des cellules immunitaires telles que lymphocytes, macrophages, mastocytes, cellules dendritiques et/ou des cellules adipeuses et/ou des annexes cutanées, comme des poils, des cheveux, des glandes sébacées.

La présente demande concerne également un procédé de préparation du modèle de peau sensible reconstituée tel que décrit ci-dessus. Le procédé de préparation du modèle de peau sensible de la demande comprend les étapes de :

a) obtention d'un modèle de peau reconstituée à partir d'un échantillon cutané d'un sujet ; et
b) traitement dudit modèle de peau reconstituée de l'étape a) par un stress exogène.

Les inventeurs ont plus particulièrement montré que l'application d'acide lactique permet d'obtenir un modèle de peau reconstituée qui reproduit les caractéristiques de la peau sensible. Notamment, l'application d'acide lactique entraine une expression plus importante de marqueurs de l'inflammation sans compromettre la structure histologique de la peau, ni affecter la viabilité cellulaire cutanée. Le stress exogène de la demande sera donc préférentiellement une application d'acide lactique.

Selon un mode de réalisation particulier, le procédé de préparation du modèle de l'invention comprend les étapes de :

a) obtention d'un modèle de peau reconstituée à partir d'un échantillon cutané d'un sujet ; et
b) mise en contact dudit modèle de peau reconstituée de l'étape a) avec de l'acide lactique.

Avantageusement, ledit échantillon cutané est tel que défini ci-dessus.

L'acide lactique pourra être utilisé à toute concentration permettant de maintenir la structure générale de la peau tout en induisant l'expression des marqueurs de l'inflammation. De façon préférée, la concentration d'acide lactique utilisée est comprise entre 0,05 % et 5 %, préférentiellement entre 0,1 % et 2,5 %, plus préférentiellement entre 0,25 % et 1,25 %, notamment entre 0,4 % et 0,8 %. L'acide lactique pourra être utilisé en solution, à toute concentration permettant de maintenir la structure générale de la peau tout en induisant l'expression des marqueurs de l'inflammation. De façon préférée, la concentration en acide lactique dans la solution utilisée sera comprise entre 0,05 % et 5 %, en poids d'acide lactique/volume de solution. De façon plus préférée, l'acide lactique sera utilisé à une concentration comprise entre 0,1 % et 2,5 %, de façon encore plus préférée, à une concentration comprise entre 0,25 % et 1,25 %, de façon encore plus préférée, à une concentration comprise entre 0,4 % et 0,8 %. Selon un aspect encore plus préféré, l'acide lactique est utilisé à une concentration de 0,6 %, en poids d'acide lactique/volume de solution.

Selon un aspect particulier, ledit sujet est un adulte. Dans ce cas, le modèle de peau de la demande est un modèle de peau d'adulte. Selon un autre aspect, ledit sujet est un enfant. Dans ce cas, le modèle de peau de la demande est un modèle de peau d'enfant.

Le modèle de peau sensible reconstituée obtenu par le procédé de la demande est tel que défini ci-dessus. Ce modèle est particulièrement avantageux car il reproduit fidèlement les caractéristiques de la peau sensible telles qu'elles ressortent de l'étude clinique conduite par les inventeurs.

Selon un autre aspect, le procédé de la divulgation comprend les étapes de :

a) obtention d'un modèle de peau reconstituée à partir d'un échantillon cutané d'un sujet ;
b) mise en contact dudit modèle de peau reconstituée de l'étape a) avec de l'acide lactique ; et
c) mesure du niveau d'expression d'au moins un marqueur biologique.

Avantageusement, ledit marqueur biologique est tel que défini ci-dessus.

Avantageusement, le niveau d'expression dudit marqueur biologique est mesuré par les méthodes de mesure telles que définies ci-dessus.

Selon un aspect particulier, l'étape c) du procédé de la demande comprend la mesure du niveau d'expression d'au moins un marqueur biologique sélectionné dans le groupe des marqueurs de l'inflammation tels que définis plus haut, des marqueurs de la fonction barrière tels que définis plus haut et des marqueurs de l'immunité innée et de défense tels que définis plus haut.

Selon un autre aspect, le procédé de la demande comprend les étapes de :

a) obtention d'un modèle de peau reconstituée à partir d'un échantillon cutané d'un sujet ;
b) mise en contact dudit modèle de peau reconstituée de l'étape a) avec de l'acide lactique ; et
c) mesure du niveau d'expression d'une combinaison de marqueurs biologiques.

Ladite combinaison selon la divulgation comprend ou consiste en :

- au moins un marqueur de l'inflammation cutanée et au moins un marqueur de la barrière tels que définis plus haut ; ou
- au moins un marqueur de l'inflammation cutanée et au moins un marqueur de l'immunité, tels que définis plus haut ; ou
- au moins un marqueur de la barrière et au moins un marqueur de l'immunité, tels que définis plus haut.

Dans un exemple particulier, ladite combinaison comprend au moins un marqueur de l'inflammation cutanée et au moins un marqueur de la barrière et au moins un marqueur de l'immunité, tels que définis plus haut.

L'utilisation de combinaisons de marqueurs comprenant au moins un marqueur de chacun des différents types indiqués plus haut est particulièrement avantageuse.

Dans un autre exemple, le procédé de la demande peut comprendre une ou plusieurs étapes intermédiaires entre l'obtention du modèle de peau reconstituée et la mesure de l'expression du marqueur biologique, lesdites étapes correspondant à l'extraction à partir dudit modèle de peau reconstituée d'un échantillon lipidique, d'un échantillon de NMF, d'un échantillon d'ARNm (ou de l'ADNc correspondant) ou d'un échantillon de protéine. Celui-ci peut ensuite être directement utilisé pour mesurer l'expression du marqueur. La préparation ou l'extraction d'ARNm (ainsi que la rétro transcription de celui-ci en ADNc), de protéines, de lipides ou de NMF à partir d'un échantillon de cellules de peau ne sont que des procédures de routine bien connues de l'homme du métier.

En ce qui concerne certains marqueurs lipidiques tels que la prostaglandine E, il peut même ne pas être nécessaire de préparer un échantillon lipidique. En effet, ce marqueur est sécrété dans le milieu de culture. Il est alors aisé pour l'homme du métier de doser la prostaglandine E2 à partir dudit milieu de culture. Plusieurs méthodes pour doser et quantifier la prostaglandine E2 ont ainsi été décrites dans l'art, dont en particulier des méthodes ELISA. Une telle méthode est ainsi détaillée dans la partie expérimentale de la présente demande et l'homme du métier pourra se reporter à celle-ci. Il est en outre à noter que des kits sont disponibles commercialement pour le dosage de la prostaglandine E2 (par exemple, chez CissBio Assays ou chez Pierce).

Selon un aspect particulier, le procédé de la divulgation comprend en outre une étape d) de comparaison du niveau d'expression du marqueur biologique de l'étape c) avec un niveau d'expression de référence.

Avantageusement, ledit niveau d'expression de référence est tel que défini ci-dessus. Avantageusement, ladite comparaison du niveau d'expression du marqueur biologique de l'étape c) avec un niveau d'expression de référence est telle que définie ci-dessus.

La divulgation présente l'avantage de permettre l'isolement et la caractérisation aisés d'agents actifs, de matières premières cosmétiques et/ou de formulations cosmétiques. Il est ainsi possible de déterminer aisément si un actif ou une formulation sont efficaces pour traiter la peau sensible ou s'ils sont bien tolérés par la peau sensible. Le modèle de peau sensible de la divulgation permet ainsi de déterminer de façon précise quels actifs ont un effet avantageux sur la prévention ou le traitement de la peau sensible. Les procédés de la divulgation sont aussi adaptés à l'évaluation de l'activité de formulations.

Par exemple, la présente demande divulgue une méthode d'évaluation de l'efficacité *in vitro* d'un actif ou d'une formulation pour prévenir ou traiter la peau sensible, ladite méthode comprenant la détermination du niveau d'expression et/ou d'activation d'au moins un marqueur biologique tel que défini plus haut.

Selon un aspect particulier, la méthode ici divulguée comprend les étapes suivantes :

a) mettre en contact ledit actif ou ladite formulation avec le modèle de peau sensible divulgué ;
b) mesurer le niveau d'expression d'au moins un marqueur biologique divulgué dans le modèle de peau de l'étape a) ; et
c) évaluer l'efficacité dudit actif ou de ladite formulation en fonction du niveau de l'étape b).

L'invention a donc aussi pour objet une méthode d'évaluation de l'efficacité *in vitro* d'un actif ou d'une formulation cosmétique pour prévenir ou traiter la peau sensible, ladite méthode comprenant les étapes suivantes :

a) mettre en contact ledit actif ou ladite formulation avec le modèle de peau sensible de l'invention ;
b) mesurer le niveau d'expression d'au moins un marqueur biologique de l'invention dans le modèle de peau de l'étape a) ; et
c) évaluer l'efficacité dudit actif ou de ladite formulation cosmétique en fonction du niveau de l'étape b).

Par « l'efficacité d'une formulation ou d'un actif pour prévenir ou traiter la peau sensible », on entend au sens de la présente demande la capacité de la formulation ou de l'actif à annuler ou diminuer les effets liés à la peau sensible. La prévention s'entend dans le cas présent d'un traitement qui est administré avant le développement des effets de la peau sensible, tandis que la réduction correspond à un traitement qui est administré une fois que les effets de la peau sensible sont apparus.

L'évaluation de l'étape c) se fera avantageusement en comparant le niveau d'expression de l'étape b) avec un niveau d'expression de référence. Par exemple, le modèle de peau sensible qui n'a pas été traité par l'actif ou la formulation peut être utilisé comme témoin. Dans ce cas, le niveau d'expression du marqueur biologique de la divulgation est mesuré dans ledit modèle de peau sensible avant et après avoir été mis en contact avec l'actif ou la formulation cosmétique. Le niveau de d'expression du marqueur biologique de la divulgation peut aussi être comparé avec celui mesuré dans une peau normale.

L'actif ou la formulation sont efficaces pour prévenir ou traiter la peau sensible si ledit actif ou ladite formulation module l'expression du marqueur biologique de la divulgation de telle façon qu'après traitement, celle-ci soit plus semblable à celle dudit marqueur dans une peau normale. Par exemple, un actif ou une formulation efficace entraine une réduction de l'expression des marqueurs de l'inflammation et/ou une augmentation de l'expression des marqueurs de la barrière et/ou une augmentation de l'expression des marqueurs de défense et de l'immunité innée par rapport au modèle de peau sensible non traité.

Le modèle de peau sensible de la demande permet aussi de vérifier facilement la tolérance, la dermopénétration et l'efficacité d'un actif ou d'une formulation. Par exemple, on peut désirer, dans certains cas, vérifier que ces actifs ou formulations sont bien tolérés et n'induisent pas l'expression accrue de marqueurs indiquant un stress, comme par exemple les marqueurs liés à l'inflammation.

Un autre aspect ici divulgué est un procédé d'évaluation de la tolérance d'un actif ou d'une formulation, comprenant les étapes suivantes :

a) mettre en contact un actif ou une formulation avec un modèle de peau sensible ;

b) mesurer le niveau d'expression d'au moins un marqueur biologique dans le modèle de peau sensible ; et

c) évaluer si ledit actif ou formulation est bien toléré par la peau sensible.

L'invention a donc aussi pour objet une méthode d'évaluation de la tolérance d'un actif ou d'une formulation cosmétique, comprenant les étapes suivantes :

a) mettre en contact un actif ou une formulation cosmétique avec le modèle de peau sensible selon l'invention ;

b) mesurer le niveau d'expression d'au moins un marqueur biologique de l'invention dans le modèle de peau de l'étape a) ; et

c) évaluer si ledit actif ou formulation cosmétique est bien toléré par la peau sensible.

L'actif est un actif qui est bien toléré par la peau sensible si ledit actif ne module pas l'expression du marqueur biologique divulgué dans la présente demande. De la même façon, la formulation cosmétique est bien tolérée si l'expression du marqueur biologique n'est pas modulée par son addition sur le modèle de peau sensible. Ladite modulation peut correspondre, selon les cas, et en particulier selon la nature du marqueur biologique, à une augmentation ou à une diminution de l'expression dudit marqueur. Il est possible d'utiliser n'importe lequel des marqueurs identifiés ici et décrits plus haut. En particulier, l'expression des marqueurs de l'inflammation est connue pour être augmentée quand la peau sensible est agressée. En revanche, l'expression de ces marqueurs de l'inflammation n'est pas affectée par des actifs ou des formulations bien tolérés. Par exemple, le marqueur biologique de l'étape b) est un marqueur de l'inflammation. Plus préférablement, le marqueur biologique de l'étape b) est IL1 ou IL8.

L'évaluation de l'étape c) se fera avantageusement en comparant le niveau d'expression de l'étape b) avec un niveau d'expression de référence. Par exemple, le modèle de peau sensible qui n'a pas été traité par l'actif ou la formulation peut être utilisé comme témoin. Dans ce cas, le niveau d'expression du marqueur biologique est mesuré dans ledit modèle de peau sensible avant et après avoir été mis en contact avec l'actif ou la formulation cosmétique. Le niveau de d'expression du marqueur biologique peut aussi être comparé avec celui mesuré dans une peau normale.

Le niveau d'expression de référence est de préférence le niveau d'expression dudit marqueur biologique dans un modèle de peau qui n'a pas été en contact avec l'actif ou la formulation, ce qui permet d'effectuer une comparaison significative entre le niveau d'expression de l'étape b) et ledit niveau de référence. Par exemple, le modèle de peau sensible qui n'a pas été traité par l'actif ou la formulation peut être utilisé comme témoin. Dans ce cas, le niveau d'expression du marqueur biologique est mesuré dans ledit modèle de peau sensible avant et après avoir été mis en contact avec l'agent actif ou la formulation cosmétique.

Le procédé tel que décrit peut en outre comporter une comparaison de la viabilité cellulaire dans le modèle de peau sensible traité avec l'actif ou la formulation et dans l'échantillon témoin. Dans ce cas, l'actif ou la formulation cosmétique est bien toléré par la peau sensible si la viabilité cellulaire de l'échantillon n'est pas affectée par la présence de l'agent actif ou la formulation cosmétique.

Selon un autre aspect, le procédé de la demande comprend donc une étape supplémentaire de détermination de la viabilité cellulaire dans le modèle de peau sensible traité avec l'actif ou la formulation cosmétique, de détermination de la viabilité cellulaire dans l'échantillon témoin et de comparaison entre les deux.

De nombreux tests pour déterminer la viabilité cellulaire sont disponibles pour l'homme du métier et sont couramment utilisés en cosmétologie. On citera en particulier le test MTT, décrit par exemple dans Mosman et al. (J Immunol Methods, 65(1-2) : 55-63, 1983).

Dans un autre aspect, la présente divulgation permet d'isoler des formulations ou des actifs permettant de réduire les effets de la peau sensible. La divulgation permet en particulier de distinguer les actifs ou les formulations selon leur activité pour traiter les effets de la peau sensible. La divulgation est donc particulièrement adaptée pour identifier des formulations ou des actifs appropriés à cette peau très spécifique.

Un aspect particulier ici divulgué est un procédé d'identification d'un actif ou d'une formulation pour le traitement de la peau sensible, caractérisé en ce que ledit procédé comprend les étapes suivantes :

a) mettre en contact ledit actif ou ladite formulation avec le modèle de peau sensible divulgué ;
b) mesurer le niveau d'expression d'au moins un marqueur biologique dans le modèle de peau de l'étape a) ; et
c) déterminer si ledit actif ou ladite formulation permet de traiter la peau sensible en fonction du niveau de l'étape b).

L'invention a donc également pour objet une méthode d'identification d'un actif ou d'une formulation pour le traitement de la peau sensible, caractérisé en ce que ledit procédé comprend les étapes suivantes :

a) mettre en contact ledit actif ou ladite formulation cosmétique avec le modèle de peau sensible de l'invention ;
b) mesurer le niveau d'expression d'au moins un marqueur biologique de l'invention dans le modèle de peau de l'étape a) ; et
c) déterminer si ledit actif ou ladite formulation permet de traiter la peau sensible en fonction du niveau de l'étape b).

La formulation candidate est une formulation pour le traitement de la peau sensible, si ladite formulation candidate permet de moduler l'expression d'au moins un marqueur biologique. Cette modulation peut correspondre, selon les cas, et en particulier selon la nature du marqueur biologique, à une augmentation ou à une diminution de l'expression dudit marqueur. De la même façon, l'actif candidat est un actif pour le traitement de la peau sensible, si ledit actif candidat permet de moduler l'expression d'au moins un marqueur biologique. Cette modulation peut correspondre, selon les cas, et en particulier selon la nature du marqueur biologique, à une augmentation ou à une diminution de l'expression dudit marqueur.

Par exemple, il peut être intéressant d'isoler des actifs ou des formulations minimisant les effets de la peau sensible sur les marqueurs de la défense et de l'immunité innée, ces formulations permettant de préserver la capacité de défense de l'épiderme contre les agressions. De même, il serait avantageux d'identifier des actifs ou des formulations minimisant les effets de la peau sensible sur les marqueurs de la barrière, afin de maintenir l'intégrité de la barrière cutanée. Enfin, il pourrait être désirable d'isoler des actifs ou des formulations qui n'induiraient pas les marqueurs de l'inflammation.

Dans un premier temps, la formulation ou l'actif d'intérêt est mis en contact avec le modèle de peau sensible tel que décrit. Cette mise en contact de l'actif d'intérêt avec le modèle de peau peut être faite directement. Alternativement, il pourra être avantageux de formuler l'actif d'intérêt, par exemple de façon à obtenir une composition liquide, afin de faciliter sa mise en contact avec le modèle de peau. Ainsi, par exemple, le procédé comprend en outre une étape de formulation de l'actif, notamment sous forme d'une solution liquide, en particulier aqueuse, préalablement à l'étape de mise en contact dudit actif avec un modèle de peau.

FIGURES

Figure 1 : Coloration hématoxyline / éosine des épidermes reconstruits.

Figure 2 : Immunomarquage de l'involucrine (gris clair) dans des épidermes reconstruits.

EXEMPLES

**Exemple 1. Caractérisation des aspect cellulaires, moléculaires et physiologiques de la peau sensible : Etude clinique**

Une étude clinique a été évaluée sur un panel de 97 sujets, dont 77 enfants.

Les sujets ont été interrogés à l'aide d'un questionnaire, afin de les classés en deux catégories : peau sensible ou peau normale. Les résultats de ce classement sont décrits dans le tableau 1.

Tableau 1 : Classement des différents sujets recrutés pour l'étude clinique, en fonction de leur âge.

| | Nombre de sujets (male/femelle) | |
|---|---|---|
| Groupe d'âge | Peau normale | Peau sensible |
| 3-6 mois | N = 15 (6/9) | N = 12 (8/4) |
| 6-12 mois | N = 11 (5/6) | N = 11 (4/7) |
| 24-48 mois | N = 18 (7/11) | N = 10 (6/4) |
| 18-20 ans | N = 10 (4/6) | N = 10 (4/6) |

Une évaluation clinique a été réalisée sur différentes parties du visage et du corps de chacun des sujets. Les critères SRRC (Scaling, Roughness, Redness and Cracks), comprenant l'évaluation des squames, de la rugosité, de l'érythème, et des fissures de la peau, et les critères ODS (Overall Dry Skin Score) comprenant l'évaluation de la sécheresse de la peau, ont été mesurés. Cette évaluation clinique a permis de montrer que la peau sensible est globalement plus squameuse, plus rugueuse et plus érythémateuse que la peau normale.

Enfin, cette évaluation clinique et ces mesures instrumentales ont été corrélées avec des analyses moléculaires. Les analyses moléculaires ont été effectuées sur des prélèvement biologiques non invasifs pour chacun des sujets (prélèvement par écouvillons) :

(i) Mesure des taux de cytokines IL1R1, IL1$\alpha$ et IL8 ;

(ii) Mesure des taux des acides gras libres polyinsaturés (acide arachidonique (C20 :4 AA) et acide linoléique (C18 :2 LA)).

Les analyses moléculaires ont permis de montrer que les niveaux d'expression des marqueurs de l'inflammation tels que les cytokines et les taux des acides gras libres polyinsaturés (AGPI) sont plus élevés dans la peau sensible que dans la peau normale, comme attendu.

## Exemple 2. Mise au point et validation d'un modèle de peau sensible reconstituée

### I. Matériel et Méthodes

*a. Modèle mis en œuvre - Induction d'un stress à l'acide lactique*

Des épidermes humains reconstruits ont été obtenus à partir des kératinocytes d'un donneur âgé de 19 ans selon la méthode dérivée de Poumay et al. (Arch Dermatol Res 2004 ; 296:203-11). Après 2 jours de culture en immersion, les épidermes humains reconstruits ont été cultivés à l'interface air/liquide pendant 10 jours.

Les épidermes à J10 de différenciation ont été traités topiquement par une solution d'acide lactique à 0,6% à raison de 50 μL/épiderme et incubés pendant 48 heures, avec renouvellement du traitement à 24 heures.

A la fin de l'incubation, différents paramètres ont été mesurés afin d'évaluer l'effet de l'acide lactique sur la réactivité des épidermes.

*b. Evaluation de la viabilité des épidermes*

La viabilité des épidermes a été évaluée par un test de métabolisation du MTT (n=2). La densité optique (DO) à 540 nm est proportionnelle à la quantité de cellules vivantes et à leur activité métabolique.

*c. Analyses histologiques*

Les épidermes ont été inclus en paraffine (n=2), leur morphologie a été évaluée suite à une coloration hématoxyline / éosine.

D'autre part, l'involucrine a été marquée par immunofluorescence (Alexa-488 ; marquage vert), les noyaux ont été colorés par l'iodure de propidium (marquage rouge). Le marquage involucrine a été quantifié par mesure de l'intensité de fluorescence rapportée à la surface des couches vivantes de l'épiderme.

*d. Analyses géniques*

L'expression génique de marqueurs inflammatoires et de marqueurs de l'immunité innée et de défense a été évaluée par PCR quantitative en temps réel (qRT-PCR) sur les ARNs messagers extraits des épidermes (n=2). L'expression génique des marqueurs de l'immunité innée et de défense, la bêta-défensine 2 hBD2, ou le Toll-like receptor 2, a été également évaluée de la même façon.

L'analyse d'expression génique a été réalisée en duplicat à l'aide d'un PCR array. Les gènes marqueurs inflammatoires étudiés sont présentés dans le tableau 2.

L'analyse quantitative de l'expression génique a été basée sur l'analyse des cycles seuils ; l'expression relative (ER) a été exprimée en unité arbitraire selon la formule suivante :

$$\text{ER} = (1/2\text{nombre of cycles}) \times 10^6$$

Tableau 2 : Classification des gènes étudiés

| Groupe de marqueurs | Gène | Abréviation |
|---|---|---|
| **Inflammation (composante tissulaire)** | Interleukine 1 alpha | IL1A |
| | Interleukine 1 beta | IL1B |
| | Interleukine 1 receptor antagonist | IL1RN |
| | Tumor Necrosis Factor | TNF |
| | Interleukine 8 | IL8 |
| | Prostaglandine-endoperoxide Synthase 2 (= COX2) | PTGS2 |
| | Phospholipase A2, group IIF | PLA2G2F |
| **Inflammation (composante vasculaire) : Facteurs de vascularisation** | Vascular Endothelial Growth Factor A | VEGF-A |
| | Thrombospondin 1 | THBS1 |
| | Adenylate Cyclase Activating Polypeptide 1 (PACAP) | ADCYAP1 |
| **Immunité innée et Défense** | Beta-Défensine-2 (hBD2) | DEFB4A |
| | Toll Like Receptor 2 | TLR2 |

*e. Dosages des marqueurs inflammatoires*

*i. Dosages des marqueurs inflammatoires relargués par les épidermes*

La quantité de marqueurs inflammatoires présents dans les sous-nageants de culture a été évaluée (n=6) à l'aide de kits CBA (BD Biosciences) pour l'IL-1alpha (IL1$\alpha$) et l'IL-8 ; et à l'aide kits ELISA pour le VEGF (R&D Systems) et la PGE2 (Enzo).

*ii. Evaluations des marqueurs inflammatoires intracellulaires*

Les quantités intracellulaires d'IL-1$\alpha$, IL8 et IL1-RA ont été dosées par ELISA (R&D Systems) dans les broyats des épidermes (n=2).

*iii. Dosage des Acides Gras Poly-Insaturés (AGPI)*

Un criblage des acides gras poly insaturés (AGPI) exprimés par les épidermes a été réalisé par analyse GC/MS des échantillons obtenus après broyage des épidermes (n=2). Les quantités ont été normalisées par le C16:0 (acide palmitique), représentatif de la teneur globale en lipides de l'épiderme.

II. Résultats

*a. Morphologie et viabilité des épidermes reconstruits*

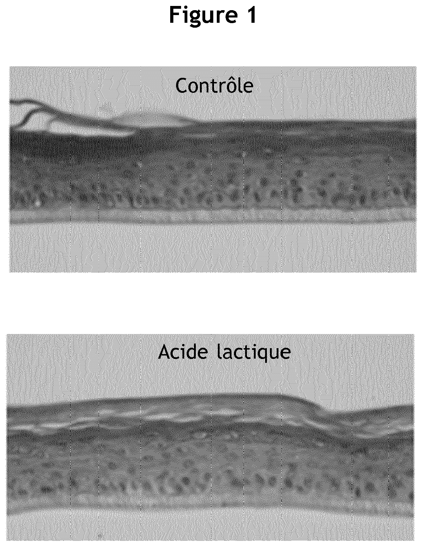

L'acide lactique a induit une diminution modérée de la viabilité des épidermes (tableau 3) et n'a pas induit d'altération majeure de leur morphologie (Figure 1).

Tableau 3 : Viabilité des épidermes reconstruits (MTT)

| Epidermes reconstruits | Viabilité |
|---|---|
| Contrôle | 100% |
| Acide lactique 0,6% | 66% |

*b. Evaluation des marqueurs géniques modulés par l'acide lactique*

L'application d'acide lactique à la surface des épidermes reconstruits a induit une stimulation des marqueurs de l'inflammation de la composante tissulaire et de la composante vasculaire, suggérant une plus grande sensibilité de la peau (tableau 4).

Tableau 4 : Expression génique dans des épidermes reconstruits (qRT-PCR)

| Groupe de marqueurs | Gènes | Contrôle | Acide lactique 0,6% |
|---|---|---|---|
| Inflammation (composante tissulaire) | IL1A | 100 | 240 |
| | IL1B | 100 | 195 |
| | IL1RN | 100 | 144 |
| | TNF | 100 | 105 |
| | IL8 | 100 | 649 |
| | PTGS2 | 100 | 182 |
| | PLA2G2F | 100 | 129 |
| Inflammation (composante vasculaire) : Facteurs de vascularisation | VEGF-A | 100 | 138 |
| | THBS1 | 100 | 181 |
| | ADCYAP1 | 100 | 117 |
| Immunité innée et défense | DEFB4A | 100 | 6 |
| | TLR2 | 100 | 54 |

Le traitement par l'acide lactique des épidermes reconstruits a induit une diminution de l'expression des marqueurs de l'immunité innée et de défense (bêta-défensine 2 hBD2 - DEFB4A, Toll-like receptor 2 TLR2), suggérant une altération des capacités de défense.

*c. Expression de l'involucrine*

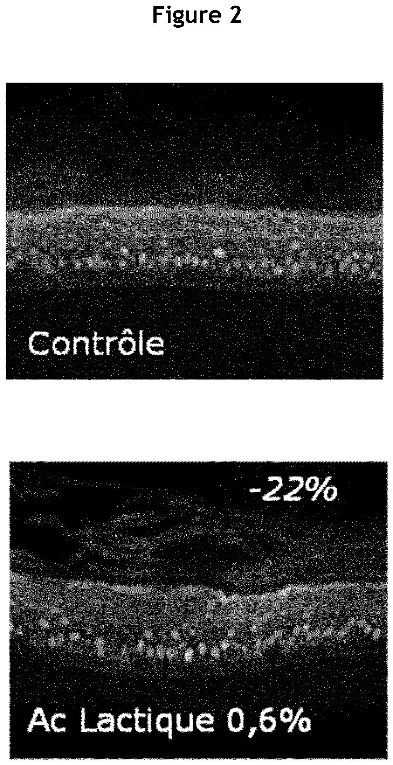

L'acide lactique a induit une diminution du niveau d'expression de l'involucrine (-22% ; Figure 2), montrant une altération de la barrière de l'épiderme.

*d. Evaluation de la réponse inflammatoire*

Le traitement à l'acide lactique a induit une augmentation de la quantité intracellulaire (tableau 4) et relarguée dans le surnageant (tableau 5) des marqueurs précoces de l'inflammation, comme les cytokines inflammatoires IL1-alpha (Interleukine 1-alpha, cytokine primaire, principal déclencheur de la cascade inflammatoire) et IL8 (Interleukine 8, chimiokine induite par synthèse de novo dans le kératinocyte stimulé).

Au niveau intracellulaire, la quantité d'IL1-RA (Interleukin 1 Receptor Antagonist, membre de la famille de l'IL1 qui est induit en réponse à une irritation, secondairement à l'IL1) a également été augmentée en réponse au traitement par l'acide lactique, tout comme le ratio IL1-RA/IL1-alpha (tableau 5).

L'équilibre du ratio IL1-RA/IL1-alpha permet de maintenir l'homéostasie cutanée. L'augmentation de ce ratio ainsi que de l'IL1-RA sont des marqueurs sensibles du niveau d'inflammation cutanée.

Le traitement à l'acide lactique a induit une induction du relargage de la PGE2 (Prostaglandine E2, marqueur lipidique de la composante tissulaire de l'inflammation) et du VEGF (Vascular Endothelial Growth Factor, facteur de croissance vasculaire et marqueur de la composante vasculaire de l'inflammation ; tableau 6).

Tableau 5 : Dosages intracellulaires des marqueurs inflammatoires (ELISA)

| | Contrôle | Acide lactique 0,6% | Evolution vs contrôle |
|---|---|---|---|
| IL8 (pg/mg de protéines) | 10,6 | 52,1 | +390% |

(suite)

|  | Contrôle | Acide lactique 0,6% | Evolution vs contrôle |
|---|---|---|---|
| IL1-alpha (pg/mg de protéines) | 88 | 794 | +806% |
| IL1-RA (pg/mg de protéines) | 2328 | 31243 | +1242% |
| Ratio IL1-RA/IL1-alpha | 26,6 | 39,3 | +48% |

Tableau 6 : Dosages des marqueurs inflammatoires relargués (ELISA)

|  | Contrôle | Acide lactique 0,6% | Evolution vs contrôle |
|---|---|---|---|
| IL1 -alpha (pg/ml) | 7 ± 1 | 23 ± 7 | +221% ns |
| IL8 (pg/ml) | 65 ± 4 | 120 ± 18 | +84% p<0,05 |
| PGE2 (pg/ml) | 48 ± 4 | 149 ± 36 | +207% p<0,05 |
| VEGF (pg/ml) | 433 ± 17 | 1109 ± 45 | +156% p<0,001 |

*e. Analyse des Acides Gras Poly-Insaturés (AGPI)*

Le criblage des AGPI révèle un effet inducteur de l'acide lactique sur les acides gras pro-inflammatoires oméga 6 impliqués dans la voie de l'acide arachidonique (AA) et de l'acide linoléique (LA) ainsi que sur les acides gras pro-inflammatoires oméga 9 impliqués dans la voie de l'acide oléique (C18 :1; tableau 7).

Tableau 7 : Dosage des acides gras polyinsaturés (AGPI) dans des épidermes reconstruits (GC/MS) ; quantités normalisées par C16:0

| AGPI | Contrôle | Acide lactique 0,6% | Evolution vs contrôle |
|---|---|---|---|
| C18 :1 | 0,189 | 0,226 | +20% |
| C18 :2 LA | 0,181 | 0,216 | +19% |
| C20 :4 AA | 0,035 | 0,050 | +42% |

**Conclusions**

Le traitement des modèles de peau reconstituées par l'acide lactique a induit une augmentation significative de l'expression des différents marqueurs protéiques des composantes tissulaires et vasculaires de l'inflammation, à la fois au niveau des transcrits et des protéines, ainsi que des marqueurs lipidiques de l'inflammation. Ce traitement des modèles de peau reconstituées a également induit une diminution significative de l'expression des marqueurs de l'immunité innée et de défense et des marqueurs de la barrière.

Ces données sont en corrélation avec les résultats obtenus dans l'étude clinique décrite dans la section 1 ci-dessus.

Le traitement des modèles de peau reconstituées par l'acide lactique permet donc de reproduire les caractéristiques de la peau sensible.

**Exemple 3. Evaluation de l'efficacité d'actifs dans le modèle de Peau Sensible reconstituée (présenté à l'exemple 2)**

I. Matériel et Méthodes

*a. Composés évalués - Actifs dermo-cosmétiques*

Les ingrédients décrits ci-après ont été évalués dans le modèle de Peau Sensible reconstituée obtenu selon l'exemple 2 décrit ci-dessus. Les ingrédients testés sont des actifs cosmétiques aux propriétés anti-inflammatoires reconnues.

- Le Cyclocéramide (OX100) [Laboratoires Expanscience]: testé à 10 et 100 $\mu$M

Le Cyclocéramide est un ingrédient actif obtenu par synthèse. C'est un inhibiteur de PKC ayant des propriétés anti-inflammatoires (EP 2 266 530 B1 ; WO2006/11443 A1 ; WO 03/055463 A1 ; Piccardi et al., 2005, JID, vol 124, n°4, suppl, abstract 216 ; Staquet et al., Int Arch Allergy Immunology, 2004, Apr;133(4):348-56).

- **L'extrait peptidique et osidique de Schizandra (SC) [Laboratoires Expanscience - nom commercial Sweetone®] :** testé à 0.05% et 0.1%

Cet extrait présente des propriétés anti-inflammatoires et anti-rougeurs démontrées in vitro et cliniquement (WO2011/012612 ; WO2011/012615 A3; EP2015/077795 ; Brédif et al., 2013, JID, Volume 133, Issue S1, page S162 , Abstract 953).

- **Le Dipotassium glycyrrhizinate (GLY) [Maruzen Pharmaceuticals]** : testé à 0.005% et 0.01%

Le dipotassium glycyrrhizinate est un extrait de Licorice aux propriétés anti-inflammatoires.

*b. Protocole*

Des épidermes humains reconstruits ont été obtenus à partir des kératinocytes d'un donneur âgé de 2 mois et demi selon la méthode dérivée de Poumay et al. (Arch Dermatol Res 2004; 296:203-11). Après 2 jours de culture en immersion, les épidermes humains reconstruits ont été cultivés à l'interface air/liquide pendant 10 jours.

Les épidermes à J10 de différenciation ont été traités ou non (contrôle) en systémique avec les composés à l'essai puis incubés pendant 24 heures. Après incubation (J11), le traitement à l'acide lactique à 0,6% (application topique de 50 $\mu$L/épiderme) a été réalisé et le milieu remplacé par du milieu contenant les composés à l'essai puis les épidermes ont été incubés pendant 48 heures, avec renouvellement du traitement à 24 heures (acide lactique en topique et composés à l'essai en systémique).

A la fin de l'incubation, les paramètres inflammatoires décrits ci-après ont été mesurés afin d'évaluer l'effet de l'acide lactique sur la réactivité des épidermes.

- *Dosages des marqueurs inflammatoires relargués par les épidermes (n=8)*

La quantité des médiateurs inflammatoires IL-1alpha (IL1$\alpha$), IL-8 et PGE2 présents dans les sous-nageants de culture des épidermes a été évaluée à l'aide de kits ELISA (R&D Systems).

- *Evaluations des marqueurs inflammatoires intracellulaires IL-1a, IL8 et IL1-RA (n=2)*

Protocole identique à celui décrit dans l'exemple 2.

- *Dosage des Acides Gras Poly-Insaturés (AGPI) (n=2)*

Protocole identique à celui décrit dans l'exemple 2.

II. Résultats

a. *Evaluation de la réponse inflammatoire*

Les résultats confirment les observations décrites dans l'exemple 2 démontrant l'effet de l'acide lactique sur l'induction d'une augmentation de la quantité intracellulaire (tableaux 8) et relarguée (tableau 9) des marqueurs de l'inflammation : IL8, IL1$\alpha$, IL1RA, Ratio IL1RA/IL1$\alpha$, PGE2.

Les ingrédients actifs évalués ont significativement inhibé ces marqueurs de l'inflammation :
Le Dipotassium Glycyrrhizinate (dénommé GLY dans les tableaux de résultats) a significativement inhibé l'augmentation de la quantité intracellulaire d'IL8, IL1$\alpha$ et IL1RA ainsi que le relargage d'IL1$\alpha$ et PGE2 induits par le traitement à l'acide lactique. Par ailleurs, le Dipotassium Glycyrrhizinate a également permis de maintenir le ratio IL1RA/IL1$\alpha$.

L'extrait peptidique et osidique de Schizandra (SC) a significativement inhibé la quantité intracellulaire d'IL1$\alpha$ et IL1RA, préservant également l'équilibre du ratio IL1RA/IL1$\alpha$. L'extrait de Schizandra a également inhibé l'induction du relargage de PGE2.

Le Cyclocéramide (OX100) a significativement inhibé l'augmentation intracellulaire de l'IL1RA et préservé l'équilibre du ratio IL1RA/IL1$\alpha$. De plus, l'OX100 a significativement inhibé le relargage d'IL1$\alpha$, IL8 et PGE2 induit par l'acide lactique.

Tableaux 8 : Dosages intracellulaires des médiateurs inflammatoires (ELISA) :

IL8, IL1$\alpha$, IL1RA, Ratio IL1RA/IL1$\alpha$

*En gras : Evolution vs contrôle ; En italique : Evolution vs Acide lactique*

*Analyse de Variance à un facteur suivie d'un test de Tukey*

| IL8 | IL8 Moy ± SD (pg/mg de protéines) | Evolution |
|---|---|---|
| Contrôle | 37,23 ± 6,484 | |
| Ac Lactique 0,6% | 79,20 ± 7,715 | **+113% p<0,01** |
| GLY 0.01% | 50,17 ± 3,366 | *-37% p<0,05* |
| GLY 0.005% | 78,39 ± 6,350 | *-1% ns* |
| SC 0.1% | 53,00 ± 9,291 | *-33% ns* |
| SC 0.05% | 70,57 ± 3,825 | *-11% ns* |
| OX100 100µM | 56,95 ± 7,460 | *-28% ns* |
| OX100 10µM | 76,24 ± 8,309 | *-4% ns* |

| IL1 alpha | IL1-alpha Moy ± SD (pg/mg de protéines) | Evolution |
|---|---|---|
| Contrôle | 277,0 ± 46,67 | |
| Ac Lactique 0,6% | 1044 ± 123,0 | **+277% p<0,001** |
| GLY 0.01% | 587,0 ± 63,64 | *-44% p<0,01* |
| GLY 0.005% | 987,5 ± 101,1 | *-5% ns* |
| SC 0.1% | 448,0 ± 76,37 | *-57% p<0,01* |
| SC 0.05% | 984,0 ± 116,0 | *-6% ns* |
| OX100 100µM | 713,5 ± 113,8 | *-32% ns* |
| OX100 10µM | 919,5 ± 53,03 | *-12% ns* |

| IL1RA | IL1RA<br><br>Moy ± SD (pg/mg de protéines) | Evolution |
|---|---|---|
| Contrôle | 5217 ± 688 | |
| Ac Lactique 0,6% | 25750 ± 1974 | +394% p<0,001 |
| GLY 0.01% | 11244 ± 1341 | -56% p<0,001 |
| GLY 0.005% | 25195 ± 1305 | -2% ns |
| SC 0.1% | 8605 ± 1527 | -67% p<0,001 |
| SC 0.05% | 22739 ± 1312 | -12% ns |
| OX100 100µM | 14581 ± 1989 | -43% p<0,001 |
| OX100 10µM | 23566 ± 1624 | -8% ns |

| Ratio IL1RA/IL1$\alpha$ | Ratio IL1RA / IL1-alpha<br><br>Moy ± SD (pg/mg de protéines) | Evolution |
|---|---|---|
| Contrôle | 18,93 ± 0,7142 | |
| Ac Lactique 0,6% | 24,73 ± 1,011 | +31% p<0,001 |
| GLY 0.01% | 19,14 ± 0,2121 | -23% p<0,001 |
| GLY 0.005% | 25,58 ± 1,287 | +3% ns |
| SC 0.1% | 19,20 ± 0,1556 | -22% p<0,001 |
| SC 0.05% | 23,20 ± 1,407 | -6% ns |
| OX100 100µM | 20,46 ± 0,4808 | -17% p<0,01 |
| OX100 10µM | 25,63 ± 0,2758 | +4% ns |

Tableau 9 : Dosages des médiateurs inflammatoires relargués (ELISA)

*En gras : Evolution vs contrôle ; En italique : Evolution vs Acide lactique*

*Analyse de Variance à un facteur suivie d'un test de Tukey*

| | IL1-alpha | | IL8 | | PGE2 | |
|---|---|---|---|---|---|---|
| | Moyenne ± Ecart-type (pg/ml) | Evolution | Moyenne ± Ecart-type (pg/ml) | Evolution | Moyenne ± Ecart-type (pg/ml) | Evolution |
| Contrôle | 11 ± 2 | | 78 ± 10 | | 29 ± 22 | |
| Acide Lactique 0,6% | 68 ± 34 | **+511% p<0,001** | 209 ± 40 | **+169% p<0,001** | 197 ± 79 | **+584% p<0,001** |
| GLY 0,01% | 35 ± 16 | *-49% p<0,05* | 244 ± 138 | *+17% ns* | 137 ± 83 | *-31% ns* |
| GLY 0,005% | 28 ± 10 | *-59% p<0,01* | 211 ± 41 | *+1% ns* | 94 ± 36 | *-52% p<0,05* |
| SC 0,1% | 91 ± 68 | *+34% ns* | 293 ± 49 | *+40% ns* | 127 ± 56 | *-36% ns* |
| SC 0,05% | 84 ± 59 | *+24% ns* | 261 ± 50 | *+25% ns* | 113 ± 39 | *-43% p<0,05* |
| OX100 à 100μM | 31 ± 19 | *-54% p<0,05* | 146 ± 24 | *-30% p<0,01* | 100 ± 48 | *-49% p<0,01* |
| OX100 à 10μM | 27 ± 13 | *-61% p<0,01* | 175 ± 30 | *-16% ns* | 98 ± 28 | *-50% p<0,01* |

*b. Analyse des Acides Gras Poly-Insaturés (AGPI)*

Le criblage des AGPI a permis de confirmer l'effet inducteur de l'acide lactique sur les acides gras pro-inflammatoires oméga 6 (C18:2 ; C20:4) et oméga 9 (C18:1) (tableau 10).

Les ingrédients actifs évalués ont montré un effet inhibiteur de l'induction des acides gras pro-inflammatoires.

Tableau 10 : Dosage des acides gras polyinsaturés (AGPI) dans les épidermes reconstruits (GC/MS) ; quantités normalisées pas C16:0

*En gras : Evolution vs contrôle ; En italique : Evolution vs Acide lactique*

*Analyse de Variance à un facteur suivie d'un test de Tukey*

| | C18:1 | | C18:2 LA | | C20:4 AA | |
|---|---|---|---|---|---|---|
| | Moyenne ± SD | Evolution | Moyenne ± SD | Evolution | Moyenne ± SD | Evolution |
| **Contrôle** | 0,1850 ± 0,02404 | | 0,09100 ± 0,01697 | | 0,0270 ± 0,008485 | |
| **Ac Lactique 0,6%** | 0,3880 ± 0,07212 | **+110% p<0,01** | 0,2130 ± 0,04808 | **+134% p<0,05** | 0,0575 ± 0,01626 | **+113% ns** |
| **GLY 0.01%** | 0,2680 ± 0,01697 | *-31% ns* | 0,1440 ± 0,01273 | *-32% ns* | 0,0395 ± 0,009192 | *-31% ns* |
| **GLY 0.005%** | 0,2785 ± 0,02475 | *-28% ns* | 0,1600 ± 0,01414 | *-25% ns* | 0,0435 ± 0,009192 | *-24% ns* |
| **SC 0.1%** | 0,2245 ± 0,01202 | *-42% p<0,05* | 0,1190 ± 0,01273 | *-44% ns* | 0,0330 ± 0,002828 | *-43% ns* |
| **SC 0.05%** | 0,2520 ± 0,02404 | *-35% ns* | 0,1400 ± 0,01697 | *-34% ns* | 0,0350 ± 0,007071 | *-39% ns* |
| **OX100 100µM** | 0,2065 ± 0,007778 | *-47% p<0,05* | 0,0970 ± 0,008485 | *-54% p<0,05* | 0,0265 ± 0,002121 | *-54% ns* |
| **OX100 10µM** | 0,2795 ± 0,03323 | *-28% ns* | 0,1515 ± 0,02192 | *-29% ns* | 0,0380 ± 0,005657 | *-34% ns* |

*c. Conclusion*

Dans le modèle de peau sensible sur épiderme reconstitué, les ingrédients actifs évalués ont modulé la réponse inflammatoire induite par l'acide lactique, par une action sur les médiateurs inflammatoires produits par les cellules cutanées (cytokines et prostaglandine) ainsi que sur les voies inflammatoires dérivées des acides gras oméga 6 et oméga 9.

Ces résultats confirment la validité du modèle de peau sensible. Ces résultats confirment également la validité du modèle de peau sensible pour une utilisation dans des méthodes d'évaluation de l'efficacité d'actifs cosmétiques pour prévenir ou traiter la peau sensible.

**Revendications**

1. Modèle de peau sensible reconstituée, ledit modèle étant susceptible d'être obtenu par un procédé comprenant des étapes de :

   a) obtention d'un modèle de peau reconstituée à partir d'un échantillon cutané d'un sujet ; et
   b) mise en contact dudit modèle de peau reconstituée de l'étape a) avec de l'acide lactique, la concentration d'acide lactique utilisée étant comprise entre 0,05 % et 5 % poids d'acide lactique/volume de solution;

   ledit modèle exprimant au moins un marqueur biologique choisi dans le groupe des marqueurs de l'inflammation, des marqueurs de la barrière et des marqueurs de défense ; et dans lequel :

   (i) l'expression du marqueur biologique choisi dans le groupe des marqueurs de l'inflammation est plus élevée que dans la peau normale, et/ou
   (ii) l'expression du marqueur biologique choisi dans le groupe des marqueurs de la barrière est moins élevée que dans la peau normale ; et/ou
   (iii) l'expression du marqueur biologique choisi dans le groupe des marqueurs de défense est moins élevée que dans la peau normale.

2. Modèle de peau sensible reconstituée selon la revendication 1, dans lequel la concentration d'acide lactique utilisée est comprise entre 0,1 % et 2,5 %, préférentiellement entre 0,25 % et 1,25 %, plus préférentiellement entre 0,4 % et 0,8 %.

3. Modèle de peau sensible reconstituée selon l'une quelconque des revendications 1 ou 2, **caractérisé en ce que** la concentration d'acide lactique utilisée est de 0,6 %.

4. Modèle de peau sensible reconstituée selon l'une quelconque des revendications 1 à 3, **caractérisé en ce que** lesdits marqueurs de l'inflammation sont choisis parmi IL1$\alpha$, ILR1A, IL8, les acides gras polyinsaturés $\omega$-6, les acides gras polyinsaturés $\omega$-9, la prostaglandine E2, PLA2G2F, MGST1 et VEGF-A.

5. Modèle de peau sensible reconstituée selon l'une quelconque des revendications 1 à 4, **caractérisé en ce que** lesdits marqueurs de la barrière sont choisis parmi l'involucrine, un céramide choisi dans le groupe des céramides CER 1 à 9, et le NMF.

6. Modèle de peau sensible reconstituée selon l'une quelconque des revendications 1 à 5, **caractérisé en ce que** lesdits marqueurs de défense sont choisis parmi la bêta-défensine 2, la protéine S100A7 et le Toll-like receptor 2.

7. Modèle de peau sensible reconstituée selon l'une quelconque des revendications 1 à 6, **caractérisé en ce que** le modèle de peau reconstituée de l'étape a) est choisi parmi les cultures de cellules cutanées en suspension, les cultures de cellules cutanées en monocouche, les cultures de cellules cutanées en bicouche, les cultures de peaux reconstruites et les cultures de muqueuses reconstruites.

8. Modèle de peau sensible reconstituée selon la revendication 7, **caractérisé en ce que** les cellules dudit modèle proviennent d'un explant de tissu cutané ou de cellules souches différenciées en cellules cutanées.

9. Modèle de peau sensible reconstituée l'une quelconque des revendications 1 à 8, **caractérisé en ce que** ledit modèle comprend au moins des fibroblastes ou des kératinocytes.

10. Procédé de préparation d'un modèle de peau sensible reconstituée selon l'une quelconque des revendications 1 à 9, comprenant les étapes de :

    a) obtention d'un modèle de peau reconstituée à partir d'un échantillon cutané d'un sujet ; et
    b) mise en contact dudit modèle de peau reconstituée de l'étape a) avec de l'acide lactique, la concentration d'acide lactique utilisée étant comprise entre 0,05 % et 5 % poids d'acide lactique/volume de solution.

11. Méthode d'évaluation de l'efficacité *in vitro* d'un actif ou d'une formulation cosmétique pour prévenir ou traiter la peau sensible, ladite méthode comprenant les étapes suivantes :

a) mettre en contact ledit actif ou ladite formulation cosmétique avec le modèle de peau sensible selon l'une quelconque des revendications 1 à 9 ;

b) mesurer le niveau d'expression d'au moins un marqueur biologique défini dans l'une quelconque des revendications 1 à 6 dans le modèle de peau de l'étape a) ; et

c) évaluer l'efficacité dudit actif ou de ladite formulation cosmétique en fonction du niveau de l'étape b).

**12.** Méthode d'évaluation de la tolérance d'un actif ou d'une formulation cosmétique, comprenant les étapes suivantes :

a) mettre en contact ledit actif ou ladite formulation cosmétique avec le modèle de peau sensible selon l'une quelconque des revendications 1 à 9 ;

b) mesurer le niveau d'expression d'au moins un marqueur biologique défini dans l'une quelconque des revendications 1 à 6 dans le modèle de peau de l'étape a) ; et

c) évaluer si ledit actif ou formulation cosmétique est bien toléré par la peau sensible.

**13.** Méthode d'identification d'un actif ou d'une formulation pour le traitement de la peau sensible, **caractérisé en ce que** ledit procédé comprend les étapes suivantes :

a) mettre en contact ledit actif ou ladite formulation cosmétique avec le modèle de peau sensible selon l'une quelconque des revendications 1 à 9 ;

b) mesurer le niveau d'expression d'au moins un marqueur biologique défini dans l'une quelconque des revendications 1 à 6 dans le modèle de peau de l'étape a) ; et

c) déterminer si ledit actif ou ladite formulation permet de traiter la peau sensible en fonction du niveau de l'étape b).

## Patentansprüche

**1.** Modell rekonstituierter empfindlicher Haut, wobei das Modell durch ein Verfahren erhaltbar ist, das die folgenden Schritte umfasst:

a) Erhalten eines Modells rekonstituierter Haut aus einer Hautprobe eines Probanden; und

b) Inkontaktversetzen des Modells rekonstituierter Haut von Schritt a) mit Milchsäure, wobei die verwendete Milchsäurekonzentration zwischen 0,05% und 5% Milchsäuregewicht / Lösungsvolumen liegt;

wobei das Modell mindestens einen biologischen Marker exprimiert, der aus der Gruppe der Entzündungsmarker, der Barrieremarker und der Verteidigungsmarker ausgewählt ist;

und wobei:

(i) die Expression des biologischen Markers, der aus der Gruppe der Entzündungsmarker ausgewählt ist, höher als in der normalen Haut ist, und/oder

(ii) die Expression des biologischen Markers, der aus der Gruppe der Barrieremarker ausgewählt ist, niedriger als in der normalen Haut ist; und/oder

(iii) die Expression des biologischen Markers, der aus der Gruppe der Verteidigungsmarker ausgewählt ist, niedriger als in der normalen Haut ist.

**2.** Modell rekonstituierter empfindlicher Haut nach Anspruch 1, wobei die verwendete Milchsäurekonzentration zwischen 0,1% und 2,5%, vorzugsweise zwischen 0,25% und 1,25%, vorzugsweiser zwischen 0,4% und 0,8% liegt.

**3.** Modell rekonstituierter empfindlicher Haut nach einem der Ansprüche 1 oder 2, **dadurch gekennzeichnet, dass** die verwendete Milchsäurekonzentration 0,6% beträgt.

**4.** Modell rekonstituierter empfindlicher Haut nach einem der Ansprüche 1 bis 3, **dadurch gekennzeichnet, dass** die Entzündungsmarker aus IL1$\alpha$, ILR1A, IL8, den mehrfach ungesättigten Fettsäuren w-6, den mehrfach ungesättigten Fettsäuren $\omega$-9, Prostaglandin E2, PLA2G2F, MGST1 und VEGF-A ausgewählt sind.

**5.** Modell rekonstituierter empfindlicher Haut nach einem der Ansprüche 1 bis 4, **dadurch gekennzeichnet, dass** die Barrieremarker aus Involucrin, einem Ceramid, ausgewählt aus der Gruppe der Ceramide CER 1 bis 9, und NMF ausgewählt sind.

6. Modell rekonstituierter empfindlicher Haut nach einem der Ansprüche 1 bis 5, **dadurch gekennzeichnet, dass** die Verteidigungsmarker aus Beta-Defensin 2, dem Protein S100A7 und dem Toll-like Receptor 2 ausgewählt sind.

7. Modell rekonstituierter empfindlicher Haut nach einem der Ansprüche 1 bis 6, **dadurch gekennzeichnet, dass** das Modell rekonstituierter Haut von Schritt a) aus den Hautzellkulturen in Suspension, den einlagigen Hautzellkulturen, den zweilagigen Hautzellkulturen, den rekonstruierten Hautkulturen und den rekonstruierten Schleimhautkulturen ausgewählt sind.

8. Modell rekonstituierter empfindlicher Haut nach Anspruch 7, **dadurch gekennzeichnet, dass** die Zellen des Modells aus einem Hautgewebeexplantat oder aus in Hautzellen differenzierten Stammzellen stammen.

9. Modell rekonstituierter empfindlicher Haut nach einem der Ansprüche 1 bis 8, **dadurch gekennzeichnet, dass** das Modell mindestens Fibroblasten oder Keratinozyten umfasst.

10. Verfahren zur Herstellung eines Modells rekonstituierter empfindlicher Haut nach einem der Ansprüche 1 bis 9, umfassend die folgenden Schritte:

   a) Erhalten eines Modells rekonstituierter Haut aus einer Hautprobe eines Probanden; und
   b) Inkontaktversetzen des Modells rekonstituierter Haut von Schritt a) mit Milchsäure, wobei die verwendete Milchsäurekonzentration zwischen 0,05% und 5% Milchsäuregewicht / Lösungsvolumen liegt.

11. Methode zur Bewertung der Wirksamkeit *in vitro* eines Wirkstoffs oder einer kosmetischen Formulierung zum Schutz oder zur Behandlung der empfindlichen Haut, wobei die Methode die folgenden Schritte umfasst:

   a) Inkontaktversetzen des Wirkstoffs oder der kosmetischen Formulierung mit dem Modell empfindlicher Haut nach einem der Ansprüche 1 bis 9;
   b) Messen des Expressionsniveaus mindestens eines biologischen Markers nach einem der Ansprüche 1 bis 6 in dem Hautmodell von Schritt a); und
   c) Bewerten der Wirksamkeit des Wirkstoffs oder der kosmetischen Formulierung in Abhängigkeit vom Niveau von Schritt b).

12. Methode zur Bewertung der Verträglichkeit eines Wirkstoffs oder einer kosmetischen Formulierung, umfassend die folgenden Schritte:

   a) Inkontaktversetzen des Wirkstoffs oder der kosmetischen Formulierung mit dem Modell empfindlicher Haut nach einem der Ansprüche 1 bis 9;
   b) Messen des Expressionsniveaus mindestens eines biologischen Markers nach einem der Ansprüche 1 bis 6 in dem Hautmodell von Schritt a); und
   c) Bewerten, ob der Wirkstoff oder die kosmetische Formulierung von der empfindlichen Haut gut vertragen wird.

13. Methode zur Identifizierung eines Wirkstoffs oder einer Formulierung zur Behandlung der empfindlichen Haut, **dadurch gekennzeichnet, dass** das Verfahren die folgenden Schritte umfasst:

   a) Inkontaktversetzen des Wirkstoffs oder der kosmetischen Formulierung mit dem Modell empfindlicher Haut nach einem der Ansprüche 1 bis 9;
   b) Messen des Expressionsniveaus mindestens eines biologischen Markers nach einem der Ansprüche 1 bis 6 in dem Hautmodell von Schritt a); und
   c) Bestimmen, ob der Wirkstoff oder die Formulierung erlaubt, die empfindliche Haut in Abhängigkeit vom Niveau von Schritt b) zu behandeln.

**Claims**

1. A model of reconstructed sensitive skin, said model being obtainable by a method comprising the steps of:

   a) obtaining a reconstructed skin model from a skin sample of a subject; and
   b) contacting said reconstructed skin model from step a) with lactic acid, the lactic acid concentration used being comprised between 0.05% and 5 % by weight of lactic acid /solution volume,

said model expressing at least one biological marker selected from the group of inflammation markers, barrier markers and defence markers; and wherein:

(i) the expression of the biological marker selected from the group of inflammation markers is higher than in normal skin, and/or
(ii) the expression of the biological marker selected from the group of barrier markers is lower than in normal skin; and/or
(iii) the expression of the biological marker selected from the group of defence markers is lower than in normal skin.

2. The model of reconstructed sensitive skin according to claim 1, wherein the concentration of lactic acid used is comprised between 0.1% and 2.5%, preferentially between 0.25% and 1.25%, more preferentially between 0.4% and 0.8%.

3. The model of reconstructed sensitive skin according to claim 1 or 2, **characterized in that** the concentration of lactic acid used is 0.6%.

4. The model of reconstructed sensitive skin according to any one of claims 1, to 3, **characterized in that** said inflammation markers are selected from IL1$\alpha$, ILR1A, IL8, $\omega$-6 polyunsaturated fatty acids, $\omega$-9 polyunsaturated fatty acids, prostaglandin E2, PLA2G2F, MGST1 and VEGF-A.

5. The model of reconstructed sensitive skin according to any one of claims 1 to 4, **characterized in that** said barrier markers are selected from involucrin, a ceramide selected from the group of ceramides CER 1 to 9, and NMF.

6. The model of reconstructed sensitive skin according to any one of claims 1 to 5, **characterized in that** said defence markers are selected from beta-defensin 2, protein S100A7 and toll-like receptor 2.

7. The model of reconstructed sensitive skin according to any one of claims 1 to 6, **characterized in that** the reconstituted skin model of step a) is chosen from skin cell cultures in suspension, monolayer skin cell cultures, bilayer skin cell cultures, reconstructed skin cultures and reconstructed mucous membrane cultures.

8. The model of reconstructed sensitive skin according to claim 7, **characterized in that** the cells of said model are derived from a cutaneous tissue explant or stem cells differentiated into skin cells.

9. The model of reconstructed sensitive skin according to claim 1 to 8, **characterized in that** the model comprises at least fibroblasts or keratinocytes.

10. A method of preparing a model of reconstructed sensitive skin according to any one of claims 1 to 9, comprising the steps of:

a) obtaining a reconstructed skin model from a skin sample of a subject; and
b) contacting said reconstructed skin from step a) with lactic acid, the lactic acid concentration used being comprised between 0.05% and 5% by weight of lactic acid/volume of solution.

11. A method of evaluating the in vitro efficacy of an active ingredient or a cosmetic formulation for preventing or treating sensitive skin, said method comprising the following steps:

a) contacting said active ingredient or cosmetic formulation with the sensitive skin model according to any one of claims 1 to 9;
b) measuring the expression level of at least one biological marker defined in any one of claims 1 to 6 in the skin model of step a); and
c) evaluating the efficacy of said active ingredient or cosmetic formulation according to the level in step b).

12. A method for evaluating the tolerance of an active ingredient or a cosmetic formulation, comprising the following steps:

a) contacting said active ingredient or cosmetic formulation with the sensitive skin model according to any one of claims 1 to 9;
b) measuring the expression level of at least one biological marker defined in any one of claims 1 to 6 in the skin model of step a); and

c) evaluating whether said active ingredient or cosmetic formulation is well tolerated by sensitive skin.

13. A method of identifying an active ingredient or a formulation for the treatment of sensitive skin, **characterized in that** said method comprises the following steps:

a) contacting said active ingredient or cosmetic formulation with the sensitive skin model according to any one of claims 1 to 9;

b) measuring the expression level of at least one biological marker defined in any one of claims 1 to 6 in the skin model of step a); and

c) determining whether said active ingredient or said formulation is suitable for treating sensitive skin according to the level of step b).

# Figure 1

Contrôle

Acide lactique

# Figure 2

Contrôle

-22%

Ac Lactique 0,6%

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2015104413 A
- WO 2015101677 A
- WO 2008096868 A
- WO 2014028734 A
- US 4882127 A
- US 4849077 A
- US 7556922 B
- US 6723513 B
- WO 03066896 A
- WO 2007111924 A
- US 20080020392 A
- WO 2006084132 A
- US 20090186349 A
- US 20090181860 A
- US 20090181385 A
- US 20060275782 A
- EP 1141399 B1
- EP 29678 A
- EP 285471 A
- EP 789074 A
- EP 1451302 B1
- EP 1878790 B1
- EP 1974718 A
- US 20070148771 A
- US 20100099576 A
- WO 02070729 A
- WO 2006063864 A
- WO 2006063865 A
- WO 2007064305 A
- EP 0296078 A1
- WO 01911821 A
- WO 0192322 A
- EP 2266530 B1
- WO 200611443 A1
- WO 03055463 A1
- WO 2011012612 A
- WO 2011012615 A3
- EP 2015077795 A

**Littérature non-brevet citée dans la description**

- **HOGAN et al.** *J Allergy,* 2012, vol. 2012, 901940
- **HERNANDEZ-PIGEON et al.** *The journal of investigative dermatology,* 2014, vol. 134 (3
- **RICHTERS et al.** *Skin Pharmacol Physiol.,* 2015, vol. 28 (2), 75-83
- **BERERDESCA et al.** *Int J Cosmet Sci.,* 2013, vol. 35 (1), 2-8
- **POUMAY et al.** *Archives of dermatology research,* 2004, vol. 296 (5), 203-211
- **NETZLAFF et al.** *European journal of pharmaceutics and biopharmaceutics,* 2005, vol. 60 (2), 167-178
- **WUFUER et al.** *Scientific report,* 2016, vol. 6 (1
- **CASTEX-RIZZI et al.** *British journal of dermatology,* 2014, vol. 170, 12-18
- **RICHTERS et al.** *Skin Pharmacol Pysiol,* 2015, vol. 28, 75-83
- **MISERY et al.** *Acta Derm Venereol,* 2017, vol. 97, 4-6
- **VAHLQUIST.** *Acta Derm Venereol,* 2000, vol. 80, 161
- **SHIMIZU.** *Annu Rev Pharmacol Toxicol.,* 2009, vol. 49, 123-150
- **JUNGERSTEND et al.** *Contact Dermitis,* 2008, vol. 58 (5), 255-262
- **DAYAN.** *Cosm & Toil,* 2006, vol. 121 (1), 37-44
- **FARWICK et al.** *Cosm & Toil,* vol. 124 (2), 63-72
- **MASUKAWA et al.** *J Lipid Res.,* 2009, vol. 50 (8), 1708-1719
- **FLUHR et al.** *Exp Dermatol.,* 2010, vol. 19 (6), 483-492
- **SHENDURE ; JI.** *Nat Biotechnol.,* 2008, vol. 26 (10), 1135-45
- **PIHLAK et al.** *Nat Biotechnol.,* 2008, vol. 26 (6), 676-684
- **FULLER et al.** *Nature Biotechnol.,* 2009, vol. 27 (11), 1013-1023
- **MARDIS.** *Genome Med.,* 2009, vol. 1 (4), 40
- **METZKER.** *Nature Rev. Genet.,* 2010, vol. 11 (1), 31-46
- **SULLIVAN et al.** *Arch Ophthalmol.,* 2002, vol. 120 (12), 1689-99
- **NORDBÄCK et al.** *J. High Resolut. Chromatogr.,* 1999, vol. 22, 483-486
- **TORRES et al.** *J. Chromatogr. A.,* 2005, vol. 1078, 28-34

- **DOWNING et al.** *J Invest Dermatol.,* 1981, vol. 77 (4), 358-360
- **NORDSTROM et al.** *J Invest Dermatol.,* 1986, vol. 87 (2), 260-263
- **ROBOSKY et al.** *J Lipid Res.,* 2008, vol. 49 (3), 686-692
- **O'NEILL et al.** *J Chromatogr Sci.,* 1976, vol. 14 (1), 28-36
- **VAN SMEDEN et al.** *J Lipid Res,* 2011, vol. 52 (6), 1211-1221
- **RAINVILLE et al.** *J Proteome Res.,* 2007, vol. 6 (2), 552-558
- **CASTRO-PEREZ et al.** *J Proteome Res.,* 2011, vol. 10 (9), 4281-4290
- **BOUWSTRA et al.** *J Invest Dermatol.,* 1991, vol. 97 (6), 1005-1012
- **VAN SMEDEN et al.** *J Lipid Res.,* 1991, vol. 52 (6), 1211-1221
- **GORCEA et al.** *Int J Pharm.,* 10 Novembre 2011
- **DAEHNHARDT-PFEIFFER et al.** *Skin Pharmacol Physiol.,* 2012, vol. 25 (3), 155-161
- **IWAI et al.** *J Invest Dermatol.,* 26 Avril 2012
- **CASPERS et al.** *J Invest Dermatol.,* 2001, vol. 116 (3), 434-442
- **VYUMVUHORE et al.** *J Biomed Opt.,* 2014, vol. 19 (11), 111603
- **FALCONE et al.** *Skin Pharmacol Physiol,* 2015, vol. 28, 307-317
- **PIRAUD et al.** *Rapid Commun Mass Spectrom,* 2005, vol. 19 (12), 1587-602
- **PETRITIS et al.** *Journal of Chromatography A,* 1999, vol. 833 (2), 147-155
- **HENRIKSEN et al.** *J Am SocMass Spectrom,* 2005, vol. 16 (4), 446-455
- Application of biophysics and bioengineering to the assessment of skin barrier function. Thesis. University of Bath, 2011
- **EISENBERG et al.** *Trends in Genet,* 2003, vol. 19, 362-365
- **GUENOU et al.** *Lancet,* 2009, vol. 374 (9703), 1745-1753
- **NISSAN et al.** *Proc. Natl. Acad. Sci.,* 2011, vol. 108 (36), 14861-14866
- **KRAEHENBUEHL et al.** *Nature Methods,* 2011, vol. 8, 731-736
- **ROSDY et al.** *In Vitro Toxicol.,* 1997, vol. 10 (1), 39-47
- **PONEC et al.** *J Invest Dermatol.,* 1997, vol. 109 (3), 348-355
- **PONEC et al.** *Int J Pharm.,* 2000, vol. 203 (1-2), 211-225
- **SCHMALZ et al.** *Eur J Oral Sci.,* 2000, vol. 108 (5), 442-448
- **BLACK et al.** *Tissue Eng,* 2005, vol. 11 (5-6), 723-733
- **DONGARI-BATGTZOGLOU ; KASHLEVA.** *Nat Protoc,* 2006, vol. 1 (4), 2012-2018
- **BECHTOILLE et al.** *Tissue Eng,* 2007, vol. 13 (11), 2667-2679
- **VRANA et al.** *Invest Ophthalmol Vis Sci,* 2008, vol. 49 (12), 5325-5331
- **KINICOGLU et al.** *Biomaterials,* 2009, vol. 30 (32), 6418-6425
- **AUXENFANS et al.** *Eur J Dermatol,* 2009, vol. 19 (2), 107-113
- **KINICOGLU et al.** *Biomaterials,* 2011, vol. 32 (25), 5756-5764
- **COSTIN et al.** *Altern Lab Anim,* 2011, vol. 39 (4), 317-337
- **AUXENFANS et al.** *J Tissue Eng Regen Med,* 2012, vol. 6 (7), 512-518
- **LEQUEUX et al.** *Skin Pharmacol Physiol,* 2012, vol. 25 (1), 47-55
- **MICHEL et al.** *In Vitro Cell. Dev Biol.-Animal,* 1999, vol. 35, 318-326
- **MOSMAN et al.** *J Immunol Methods,* 1983, vol. 65 (1-2), 55-63
- **POUMAY et al.** *Arch Dermatol Res,* 2004, vol. 296, 203-11
- **PICCARDI et al.** *JID,* 2005, vol. 124 (4
- **STAQUET et al.** *Int Arch Allergy Immunology,* Avril 2004, vol. 133 (4), 348-56
- **BRÉDIF et al.** *JID,* 2013, vol. 133 (S1), S162